# EUROPEAN PATENT SPECIFICATION

(11) **EP 2 095 128 B1**
(45) Date of publication and mention of the grant of the patent: **02.10.2013**
(21) Application number: 07846655.4
(22) Date of filing: 19.11.2007
(51) Int. Cl.: G01N 33/68

(54) **METHOD OF DIFFERENTIALLY DIAGNOSING DEMENTIAS**
VERFAHREN ZUR DIFFERENTIELLEN DIAGNOSE VON DEMENZKRANKHEITEN
METHODE POUR ETABLIR UN DIAGNOSTIC DIFFERENTIEL DE DEMENCES

(30) Priority: 17.11.2006 EP 06023954; 05.12.2006 EP 06025097
(43) Date of publication of application: 02.09.2009
(60) Divisional application: 11195439.2
(73) Proprietor: Friedrich-Alexander-Universität Erlangen-Nürnberg, 91054 Erlangen (DE); Georg-August-Universität Göttingen - Stiftung Öffentlichen Rechts/Universitätsmedizin, 37099 Göttingen (DE)
(72) Inventor: BIBL, Mirko, 42555 Velbert/OT Nierenhof (DE); MOLLENHAUER, Brit, 37073 Göttingen (DE); WILTFANG, Jens, 91056 Erlangen (DE); ESSELMANN, Hermann, 37308 Glasehause (DE); LEWCZUK, Piotr, 91341 Röttenbach (DE); KORNHUBER, Johannes, 90491 Nürnberg (DE)
(74) Representative: Rehberg Hüppe + Partner
(86) International application number: PCT/EP2007/009974
(87) International publication number: WO 2008/058760

(56) References cited:
- BIBL MIRKO ET AL: "CSF amyloid-beta-peptides in Alzheimer's disease, dementia with Lewy bodies and Parkinson's disease dementia." BRAIN : A JOURNAL OF NEUROLOGY MAY 2006, vol. 129, no. Pt 5, May 2006 (2006-05), pages 1177-1187, XP002469243 ISSN: 1460-2156
- WILTFANG J ET AL: "Highly conserved and disease-specific patterns of carboxyterminally truncated Abeta peptides 1-37/38/39 in addition to 1-40/42 in Alzheimer's disease and in patients with chronic neuroinflammation" JOURNAL OF NEUROCHEMISTRY, NEW YORK, NY, US, vol. 81, no. 3, May 2002 (2002-05), pages 481-496, XP002397538 ISSN: 0022-3042 cited in the application
- SCHOONENBOOM NIKI S ET AL: "Amyloid beta 38, 40, and 42 species in cerebrospinal fluid: more of the same?" ANNALS OF NEUROLOGY JUL 2005, vol. 58, no. 1, July 2005 (2005-07), pages 139-142, XP002469244 ISSN: 0364-5134
- BIBL M ET AL: "Validation of amyloid-beta peptides in CSF diagnosis of neurodegenerative dementias." MOLECULAR PSYCHIATRY JUL 2007, vol. 12, no. 7, July 2007 (2007-07), pages 671-680, XP002469245 ISSN: 1359-4184
- KAPAKI E ET AL: "CSF tau protein and beta-amyloid (1-42) in Alzheimer's disease diagnosis: discrimination from normal ageing and other dementias in the Greek population." EUROPEAN JOURNAL OF NEUROLOGY : THE OFFICIAL JOURNAL OF THE EUROPEAN FEDERATION OF NEUROLOGICAL SOCIETIES MAR 2003, vol. 10, no. 2, March 2003 (2003-03), pages 119-128, XP002426000 ISSN: 1351-5101
- GROSSMAN MURRAY ET AL: "Cerebrospinal fluid profile in frontotemporal dementia and Alzheimer's disease." ANNALS OF NEUROLOGY MAY 2005, vol. 57, no. 5, May 2005 (2005-05), pages 721-729, XP002469246 ISSN: 0364-5134

## Description

### FIELD OF THE INVENTION

The invention relates to an ex *vivo* method of differentially diagnosing dementias comprising the features of the preamble of independent claim 1. Particularly, the present invention relates to such a method which is suitable for discriminating vascular dementias and/or frontotemporal lobe degenerations from differential diagnostic relevant dementias, such as Alzheimer's disease, and dementive disorders, such as depression. The invention also relates to differentiating between vascular dementias and frontotemporal lobe degenerations. Further, the invention relates to a use of certain components in differentially diagnosing dementias according to the preamble of independent claim 15.

### ABBREVIATIONS USED

In this description, the following abbreviations are used: Aβ peptides = amyloid-beta peptides; Aβ-SDS-PAGE/immunoblot = amyloid-beta-sodium-dodecyl-sulphate-polyacrylamide-gel-electrophoresis with western immunoblot; AD = Alzheimer's disease; APP = beta-amyloid precursor protein; CCD-camera = charge coupled device camera; CSF = cerebrospinal fluid; DC = depressive control; DGN = Deutsche Gesellschaft für Neurologie; FTD = frontotemporal dementia; FTLD = frontotemporal lobe degeneration; MMSE = Mini-Mental-Status Examination; NINCDS-ADRDA = National Institute Neurological and Communicative Disorders and Stroke-Alzheimer's Disease and Related Disorders Association; SDS = sodium dodecyl sulphate; %T = percentage of acrylamide; VAD = vascular dementia.

### PRIOR ART

The differential diagnosis among the two most common forms of dementias, Alzheimer's disease (AD) and vascular dementias (VAD), is crucial, since it does influence the therapeutic strategy. Thus far, multiparametric CSF-based neurochemical dementia diagnostics (CSF-NDD) may support the differential diagnosis of AD and VAD by measuring Aβ1-42, total tau and phospho-tau.

Van Oijen et al., 2006, reported that plasma Aβ peptides may have diagnostic value in incipient AD.

Gurol et al., 2006, reported that the plasma concentration of Aβ peptide species Aβ1-40 displayed relation to the load of vascular injuries in mild cognitive impairment, AD and cerebral amyloid angiopathy. Particularly, the plasma concentration of Aβ1-40 has been found to be associated with cerebral white matter lesions independently of potential confounders.

A quintet of Aβ peptide species, i.e. Aβ 1-37/38/39/40/42, has been characterised as regular constituents of human CSF (Wiltfang et al., 2002; Lewczuk et al., 2004).

Kapaki et al., 2003, dislose the use of tau protein and Aβ1-42 concentrations measured in cerebrospinal fluid (CSF) in Alzheimer's disease diagnosis. Particularly, they propose a tau/Aβ1-42 ratio as a clinical criterion for distinguishing AD from VAD in dementia diagnosis.

In humans under the age of 65 years, FTLD is the second most common cause of dementia after Alzheimer's disease.

AD is a progressive degenerative disease of the brain that causes memory impairment and dementia manifested by confusion, visual-spatial disorientation, inability to calculate, deterioration of judgment, delusions, and hallucinations. This most common degenerative brain disease constitutes 70% of all cases of dementia in humans. Onset is usually in late middle life, and death typically ensues in 5 to 10 years.

The heterogeneous group of FTLD and AD share neurochemical and clinical features, although temporal appearance of symptoms during the course of disease and neuropathological findings differ remarkably between these two dementia syndromes.

The extracellular deposition of aggregated amyloid beta (Aβ) peptides, mainly total Aβ1-42, as amyloid-plaques is a neuropathological hallmark of Alzheimer's disease (Glenner and Wong, 1984), whereas amyloid plaques are rarely found in FTLD (Arnold et al., 2000). Aβ1-42 levels in body fluids such as cerebrospinal fluid (CSF) are significantly reduced in AD and to a lesser degree in FTLD (Hulstaert et al., 1999).

Aβ peptides are derived from a transmembrane amyloid precursor protein (APP), after cleavage by two enzymes, β- and β-secretase (Haas and Selkoe, 1993). Aβ consists predominantly of Aβ1-38, Aβ1-40 and Aβ1-42. Distinct β-secretase activities are hypothesized to be responsible for generation of either carboxy-terminal truncated (Ct-truncated) or elongated (Ct-elongated) Aβ peptides as referenced to Aβ1-40 (Citron et al., 1984). Thus, the measurement of the different Aβ peptide species in body fluids can be assumed to more adequately represent disease-specific changes of APP metabolism rather than the simple absolute Aβ1-42 levels (Wiltfang et al., 2001, Bibl et al., 2006).

To date, three sets of clinical criteria for the differential diagnosis of FTLD have been documented. The most recent set of criteria was suggested by McKhann and colleagues (McKhann et al., 2001), mainly as an aid to routine clinical diagnosis. The clinical criteria of Neary and colleagues (Neary et al., 1998) are most helpful when focusing on the three prototypic subtypes of FTLD, frontotemporal dementia (FTD), primary progressive aphasia (PPA), and semantic dementia (SD). Nevertheless, clinical diagnosis often remains crucial in a definite case and biomarkers for applicable diagnostic testing are still under intensive investigation. The constellation of reduced Aβ₁₋₄₂ in body fluids, accompanied by an elevation of tau protein levels, can be found in both FTD and AD (Blennow, 2004). Inconsistent data about the performance of these proteins as an applicable biomarker for the differential diagnosis of FTD and AD have been published to date (Sjögren et al., 2000; Riemenschneider et al., 2002). The clinical diagnosis of neurodegenerative diseases is still very difficult, specifically with respect to the differentiation of AD from FTD and FTD from PPA.

It has been suggested that Aβ forms can be used in conjunction with clinical diagnosis to support a differential diagnosis. Biomarkers to aid clinical diagnosis, such as beta amyloid 1-42 (Aβ₁₋₄₂), tau, and phosphorylated tau have been systematically investigated in FTD, but not in PPA.

### PROBLEM OF THE INVENTION

It is the problem of the present invention to provide an ex *vivo* method of differentially diagnosing vascular dementias, which displays good sensitivity and specificity so that it particularly enables discriminating vascular dementias from frontotemporal lobe degenerations or other kinds of dementia like, for example, Alzheimer's disease.

### SOLUTION

The problem of the invention is solved by a method comprising the features of independent claim 1. Preferred embodiments of this new method are defined in dependent claims 1 to 7. Claim 8 is directed to a the use of certain components in the new method. The dependent claims 9 to 15 define preferred embodiments of the new use.

### DESCRIPTION OF THE INVENTION

The inventors found the same quintet of Aβ peptide species, i.e. Aβ1-37/38/39/40/42, which have been characterised as regular constituents of human CSF before (Wiltfang et al., 2002; Lewczuk et al., 2004), in plasma. However, plasma Aβ peptides were characterised by the additional aminoterminally truncated species Aβ2-40. The latter peptide species cannot be differentiated from Aβ1-40 by currently available ELISA methods.

Further and surprisingly, the inventors found that the plasma concentrations of the individual Aβ peptide species show characteristic variations in case of vascular dementia and frontotemporal lobe degenerations.

Particularly, in case of vascular dementias, in addition to an increase of Aβ1-40 the concentrations of Aβ1-37, Aβ1-38, Aβ1-39, Aβ1-42 and Aβ2-40 are decreased. The decrease of these Aβ peptide species is characteristic for vascular dementias in that it is not displayed with other differential diagnostic relevant dementive disorders, such as Alzheimer's disease and depression. However, this decrease, except of the decrease in Aβ1-42, and the increase in Aβ1-40 are also displayed with frontotemporal lobe degenerations.

The Aβ peptide species which display particularly high decreases in their plasma concentration with vascular and frontotemporal dementias are Aβ1-37, Aβ1-38, Aβ1-42 (not with frontotemporal dementia), and Aβ2-40, the most characteristic decrease being displayed by Aβ1-38.

Thus, the present invention provides an ex *vivo* method of differentially diagnosing dementias using the carboxy-terminally truncated amyloid β peptide Aβ1-38.

In one embodiment, the method of the present invention makes use of a combination of the carboxy-terminally truncated Aβ species Aβ1-37 and Aβ1-38 as the biomarker.

In one embodiment the body fluid used in the present invention is CSF (cerebrospinal fluid).

Particularly, a concentration of Aβ1-38 is determined in a blood sample taken from the patient, and, typically, the concentration of Aβ1-38 and all other amyloid β peptide species which will be determined in the new method are plasma concentrations, i.e. the concentrations will be determined in a blood plasma sample. As plasma is a component of blood, and as a blood plasma sample is a fraction of a blood sample taken from a patient, the concentrations of the Aβ peptide species determined in the new method may also be related to the total blood sample, or they may even be directly determined in the blood sample.

From the plasma or the total blood sample, the Aβ peptides are preferably enriched using an antibody specific to Aβ peptides, and then further analysed with regard to the concentration of the individual Aβ peptide species. The use of an N-terminal specific antibody for enriching the Aβ peptides proved as being particularly successful.

The absolute concentrations of the Aβ peptide species may show a considerable variation which is not due to the occurrence of vascular dementias or frontotemporal lobe degenerations. The relative concentrations of the Aβ peptide species, however, display a much smaller variation without the occurrence of these diseases. Thus, in the new method a relative concentration of the Aβ peptide species is determined to more clearly discriminate vascular dementia and frontotemporal lobe degenerations from other kinds of dementias.

For example, the relative concentration of the Aβ peptide species can be determined as a fraction of the total Aβ peptide concentration, which will here be denoted as Aβ1-X%, or as a ratio of the absolute concentration of the at least one carboxy-terminally truncated amyloid β peptide species and the absolute concentration of the further Aβ peptide Aβ1-40 or Aβ1-42, which will here be denoted as Aβ1-X/1-40 and Aβ1-X/1-42, respectively.

To actually discriminate certain dementias, the concentration of Aβ1-38 is compared to a threshold value for Aβ1-38.

The actual threshold value for Aβ1-38 is obtainable by comparing the levels of the biomarker(s) in the patient to those in non-demented controls (like for example in non-demented depressive controls) and/or in patients with other dementias (like for example Alzheimer's disease).

In a particular embodiment of the new method, a concentration of Aβ1-38 selected from the group of concentrations consisting of
- the relative concentration of Aβ1-38determined as a fraction of the total amyloid β peptide concentration, and
- the relative concentration of Aβ1-38 determined as a ratio of the absolute concentration of Aβ1-38 and the absolute concentration of the further carboxy-terminally truncated amyloid β peptide species Aβ1-40,
above the threshold value for Aβ1-38 is taken as an indication of no vascular dementia in the patient.

If the fraction of the total Aβ peptide plasma concentration of the Aβ peptide species Aβ1-38 is determined, a suitable threshold value for diagnosing vascular dementias is in a range of 7.6 % to 8.6 %. Preferably, this threshold value is in a range of 8.29 % to 8.43 %. A threshold value within the latter range is most suited to discriminate VAD from AD.

If the ratio of the absolute plasma concentration of the Aβ peptide species Aβ1-38 and the absolute plasma concentration of the Aβ peptide species Aβ1-40 is determined, a suitable threshold value for diagnosing vascular dementias is in a range of 0.116 to 0.156. Preferably, this threshold value is about 0.136.

In another method, a concentration of Aβ1-38 selected from the group of concentrations consisting of
- the absolute concentration of Aβ1-38,
- the relative concentration of Aβ1-38 determined as a fraction of the total amyloid β peptide concentration, and
- the relative concentration of Aβ1-38 determined as a ratio of Aβ1-38 and the absolute concentration of at least one further amyloid β peptide species selected from the group consisting of Aβ1-40 and Aβ1-42,
above the threshold value for Aβ1-38 is taken as an indication of no frontotemporal lobe degeneration in the patient.

Particularly, it may vice versa be deduced that the said mammal is suffering from FTLD provided that the concentration of Aβ1-38 is lower than a threshold value for Aβ1-38 which, quantified as a percentage, is at least 20% less and preferably ranges from 20% to 40% less as compared to the concentration of the Aβ1-38 in patients with Alzheimer's disease and in non-demented controls.

Another method includes obtaining body fluid (for example, blood or CSF) samples from a mammal (for example, human) and determining the ratio of carboxy-terminal truncated Aβ peptides Aβ₁₋₃₇ to Aβ₁₋₄₀ wherein the ratio less than 0.125 indicates that the mammal is susceptible to, or has, FTLD.

Another method includes obtaining body fluid samples (for example, blood or CSF) from a mammal (for example, human) and determining the ratio of carboxy-terminal truncated Aβ peptides Aβ₁₋₃₈ to Aβ₁₋₄₀ wherein the ratio less than 0.22 indicates that the mammal is susceptible to, or has, FTLD.

In another particular embodiment of the new method, a concentration of Aβ1-38 selected from the group of concentrations consisting of
- the relative concentration of Aβ1-38 determined as a fraction of the total amyloid β peptide concentration, and
- the relative concentration of Aβ1-38 determined as a ratio of the absolute concentration of Aβ1-38 and the absolute concentration of the further carboxy-terminally truncated amyloid β peptide species Aβ1-40,
below the threshold value for Aβ1-38 is taken as an indication of a vascular dementia or a frontotemporal lobe degeneration in the patient.

Particularly in the latter embodiment, the relative concentration of the carboxy-terminally truncated amyloid β peptide species Aβ1-38 determined as a ratio of Aβ1-38 and the absolute concentration of the further amyloid β peptide species Aβ1-42, can be used to differentiate between a vascular dementia and a frontotemporal lobe degeneration in the patient, a relative concentration below the threshold value for Aβ1-38 indicating a frontotemporal lobe degeneration in the patient. Particularly, this threshold value will be in the range of 1.2 to 1.4, preferably about 1.3, if the body fluid is blood plasma, for example.

The patient from which the body fluid to be examined in the new method is obtained may be any mammal; in a particular embodiment of the present invention the mammal is a human.

In one embodiment, the methodology used for the quantification of the biomarker is a measure of immunological interaction of the biomarker with antibody(s) or antibody mimicking molecule(s), including specifically monoclonal antibodies specific to Aβ peptide biomarker. One such example of the methodology used for the quantification of the biomarker is gel electrophoresis or Aβ-SDS-PAGE/immunoblot.

In one embodiment, the biomarker may be comprised of a modified form of the carboxy-terminally truncated amyloid β peptide species of interest: the said Aβ peptide may be present in an oxidized form, in a phosphorylated form, or an N(or amino)-terminally truncated form.

The components used in the new use of components for differentially diagnosing vascular dementias according to the new method comprises all necessary components for executing the new method so far as these components are no standard components available in any well equipped laboratory. Thus the components comprises at least components for determining a concentration of the carboxy-terminally truncated amyloid β peptide Aβ1-38 in a body fluid obtained from a patient.

### SHORT DESCRIPTION OF THE DRAWINGS AND TABLES

In the following, the invention will be further described and explained by means of embodiment examples and with reference to the attached drawings and tables.
- **Fig. 1**: is a graph showing the mean and the 95% confidence interval (CI) of the ratio Aβ1-38/1-40 for three diagnostic groups of Example I consisting of DC, AD and VAD patients.
- **Fig. 2**: shows operator curves of the ratios Aβ1-38/1-40, Aβ1-42/1-40 and Aβ2-40/1-40, respectively, for the detection of VAD among all other patients in Example I.
- **Fig. 3**: belongs to Example II and shows (a) an urea-based Aβ-SDS-PAGE/immunoblot, and (b) conventional SDS-PAGE of CSF (lane 1-3) and synthetic Aβ1-37, Aβ1-38, Aβ1-39, Aβ1-40 and Aβ1-42 peptides (lane 4). Ten microliters of an unconcentrated CSF were applied to each lane. The figure shows a blot of CSF of patients with FTLD (lane 2), Non-demented disease controls (NDC) (lane 3), and AD (lane 4), respectively.
- **Fig. 4**: is a graph showing the mean and the 95% confidence interval (CI) of Aβ1-38/ Aβ1-40 for NDC, AD and FTLD in Example II.

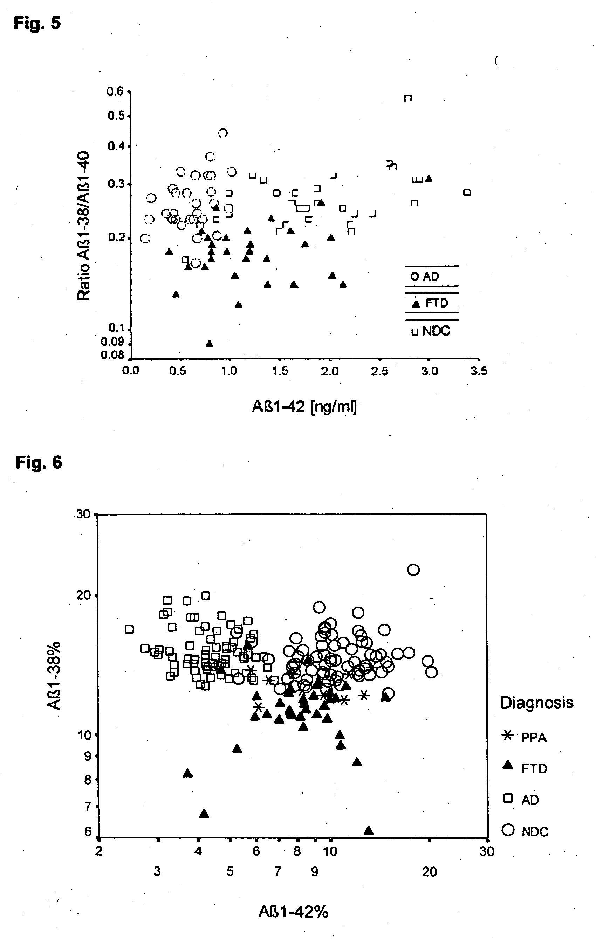

- **Fig. 5**: is a scatter plot of FTLD, AD, and NDC patients of Example II divided by the ratio of Aβ₁₋₃₈/ Aβ₁₋₄₀ and Aβ₁₋₄₂ [ng/mL]. The best cut off for differentiating FTLD from AD and NDC using Aβ₁₋₃₈/ Aβ₁₋₄₀ is 0.215.
- **Fig. 6**: is a scatter plot of FTD, AD, PPA and NDC patients of Example III divided by their percentage abundance of Aβ₁₋₄₂ and Aβ₁₋₃₈, respectively, relative to the sum of all investigated Aβ peptides.
- **Fig. 7**: is a graph of Aβ1-38% in CSF of non-demented disease controls (NDC), Alzheimer's disease (AD), Frontoremporal dementias (FTD), Primary progressive aphasia (PPA) and other dementias of Example III.
- **Fig. 8**: is a graph showing the mean and the 95% confidence interval (CI) of the ratio plasma Aβ1-38/1-40 for each diagnostic group of Example IV.

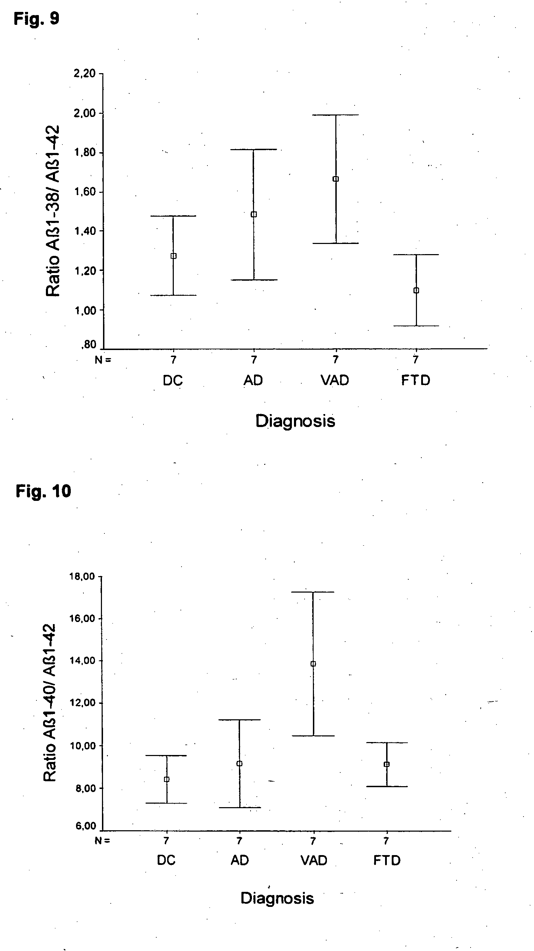

- **Fig. 9**: is a graph showing the mean and the 95% confidence interval (CI) of the ratio plasma Aβ1-38/1-42 for each diagnostic group of Example IV.
- **Fig. 10**: is a graph showing the mean and the 95% confidence interval (CI) of the ratio plasma Aβ1-40/1-42 for each diagnostic group of Example IV.
- **Table 1**: represents plasma Aβ peptide patterns, CSF Aβ1-42 and tau and demographic data of the diagnostic groups of Example I.
- **Table 2**: represents ratios of Aβ₁₋₃₈/ Aβ₁₋₄₀ for the following diagnostic groups of Example II: FTLD versus NDC and AD patients (Table 1a); FTLD versus AD (Table 2b); FTLD versus NDC (Table 2c).
- **Table 3**: represents ratio of Aβ₁₋₃₇/ Aβ₁₋₄₀ for the following diagnostic groups of Example II: FTLD versus NDC and AD patients (Table 3a); FTLD versus AD (Table 3b); FTLD versus NDC (Table 3c).
- **Table 4**: represents ratio of Aβ₁₋₄₂/ Aβ₁₋₃₈ for the following diagnostic groups of Example II: FTLD versus NDC and AD patients (Table 4a); FTLD versus AD (Table 4b); FTLD versus NDC (Table 4c) AD versus NDC (Table 4d).
- **Table 5**: represents ratio of Aβ₁₋₄₂/ Aβ₁₋₃₇ for the following diagnostic groups of Example II: FTLD versus NDC and AD patients (Table 4a); FTLD versus AD (Table 4b); FTLD versus NDC (Table 4c); AD versus NDC (Table 4d).
- **Table 6**: represents Aβ peptide patterns, Aβ₁₋₄₂ and tau in the CSF of the diagnostic groups NDC, AD and FTLD of Example II.
- **Table 7**: represents Aβ peptide patterns, Aβ₁₋₄₂ and tau in the CSF of the diagnostic groups of Example III: NDC (n=71), AD(n=71), FTD(n=36), Dementia with Lewy bodies (DLB, n=32), Other dementias (OD, n=93).
- **Table 8**: represents diagnostic accuracies of Aß peptide patterns in FTLD, AD and NDC of Example II.
- **Table 9**: represents diagnostic accuracies of Aß peptide patterns in AD, FTD, PPA among other dementias and NDC as described in Example III.
- **Table 10**: represents absolute and percentage abundances of plasma Aβ peptide species, CSF Aβ1-42, tau, and demographic data of the diagnostic groups of Example IV.

### DETAILED DESCRIPTION OF PREFERRED EMBODIMENTS OF THE INVENTION

### DEFINITIONS

In this description, the use of the terms vascular dementia and Alzheimer's disease is based on the NINCDS-AIREN and NINCDS-ARDA criteria, respectively. The application of other criteria, like for example, the ADDTC criteria is also possible and leads to comparable results.

The term "biomarker" is a characteristic that is objectively measured and evaluated as an indicator of normal biological processes, pathological processes, or pharmacological responses to a therapeutic intervention. The biomarker can for example describe a substance whose detection indicates a particular disease state. The biomarker may be a peptide that causes disease or is associated with susceptibility to disease.

The term "body fluids" includes any fluids which can be obtained from a mammalian body. Thus, the term "body fluids" also includes homogenates of any tissues and other body matter. More particularly, however, the term "body fluids" includes fluids that are normally or abnormally secreted by or excreted from the body. The respective fluids may include, but are not limited to: blood, plasma, lymph, urine, and cerebrospinal fluid, blood, plasma, and cerebrospinal fluid being of most interest here.

The term "quantifying the level" or "quantification of" may relate to absolute or relative measurement of the biomarker. Any method may be used to quantify the biomarker(s) level in the bodily fluid. For example, quantitative analysis may involve techniques such as ELISA, SDS-PAGE/immunoblot, or immunocapture-based techniques (Lewczuk et al., 2004b). Other techniques, such as immunoassays, using enzyme labeling, fluorescent labeling, and electrochemiluminescence may also be used to validate the measurement of Aβ peptides. Such techniques may involve use of polyclonal or monoclonal antibodies specific to the biomarker of interest. In one method, quantification may represent the ratio of carboxy-terminal truncated Aβ forms to the total Aβ₁₋₄₂. In another method, quantification may represent the ratio of carboxy-terminal truncated Aβ forms to the carboxy-terminal truncated Aβ₁₋₄₀. The truncated Aβ forms may also include, but are not restricted to, modified forms such as the oxidized helical form of Aβ₁₋₄₀^{ox}).

The two notations Aβ_{n-X} and Aβn-X used in this description are not intended to indicate different but the same amyloid β peptide species. Likewise, there is no difference between amyloid β peptide, beta amyloid peptide, and Aβ.

The term "antibody-mimicking molecule" refers to any molecule capable of binding to the epitope of Aβ peptide biomarker. The molecule may be a synthetic form comprising all the characteristics of a monoclonal antibody.

The term "sensitivity" refers to the capacity of a biomarker to identify a substantial percentage of mammals (for example humans) with the disease. A sensitivity of 100% would mean that a marker can identify 100% of mammals with the disease. The mathematical expression of sensitivity is the number of true positive cases of disease divided by the number of true positive cases plus the number of false negative cases.

The term "specificity" refers to the capacity of a biomarker to identify a substantial percentage of mammals (for example humans) without the disease. A specificity of 100% would mean that a mammal is healthy. The mathematical expression of specificity is number of true negative cases divided by number of true negative cases plus number of false positive cases.

The term "control" relates to healthy mammals or mammals (for example humans) suffering from mental illness that may include, but is not limited to, affective or emotional instability, behavioral dysregulation, and/or cognitive dysfunction or impairment. In one aspect of the present invention, control refers to a psychiatric cognitive complainer (PCC) and includes mammals suffering from any psychiatric disorders that may lead to cognitive deficiencies complained of by the diseased individual. This may not necessarily have to be verified cognitive testing and includes many psychiatric disorders, such as schizophrenia, depression, or sleep disorders of any kind. In another aspect of the present invention, the control refers to non-demented disease controls (NDC). In general, controls are meant to have no clinical signs of any form of dementia. In detail these are (i) Psychiatric cognitive complainers (PCC): patients suffering from any psychiatric disorder and complain about cognitive impairment during course of disease, (ii) Non-demented disease controls (NDC): patients suffering from any kind of neurologic or psychiatric diseases without any dementia syndrome , (iii) Depressive cognitive complainers (DCC): patients with cognitive complaints in the course of depression.

### EXAMPLE I

13 patients with VAD (7 men, 6 women) were investigated in comparison to AD (n=10; 7 men, 3 women) and depressive controls (n=13; 6 men, 7 women). The clinical diagnosis was made according to DSM-IV and the respective current consensus criteria (Blacker et al., 1994; Roman et al., 1993). Moreover, VAD patients were characterized by typical signs of cerebrovascular disease in brain imaging (CT or MRI), whereas AD patients displayed mild white matter lesions at maximum. AD patients were further characterized by low CSF Aβ1-42 and high tau levels using the linear function (Aβ1-42=240+1.18xtau) from a large validation study of these biomarkers (Hulstaert et al., 1999). Patients with relevant cerebrovascular disease or clear focal atrophy in brain imaging, MMSE score below 26 or anamnesis of persistent cognitive decline for at least six month were excluded from the depressive control group.

Plasma samples were taken from all patients between 2000 and 2004. Handling of the samples followed a standardized protocol according to previously published data, except for time span until freezing which ranged up to 48 hours (Lewczuk et al., 2004). For quantification, Aβ peptides were enriched from plasma using the N(or amino)-terminal specific immunoprecipitation by the monoclonal antibody 1E8, and the Aβ-SDS-PAGE/immunoblot served for analysis as published elsewhere (Wiltfang et al., 2002; Bibl et al., 2004; Lewczuk et al., 2004). Samples were run as triplicates at minimum relative to dilution series of the synthetic Aβ1-37, Aβ1-38, Aβ1-39, Aβ1-40, Aβ1-42 and Aβ2-40 on each gel. The detection sensitivity of the Aβ-SDS-PAGE/immunoblot was 0.6-1 pg (Wiltfang et al., 2002; Bibl et al., 2004), Coefficients of variations of the Aβ peptides' concentrations ranged among 14.0 and 18.5% (Lewczuk et al., 2004). Quantifications of plasma Aβ peptides were obtained from individual blots and patient groups were characterized by mean and standard deviation. These data are listed in Table 1.

Plasma peptide values are indicated as absolute (pg/ml) or percentage (amount of each peptide relative to the sum of all single investigated Aβ peptides) and ratio (amount of each peptide relative to Aβ1-40) values, respectively. Significant group differences were evaluated in the Mann-Whitney U-test. The two-sided level of significance was taken as p<0.05. Receiver operating characteristic curve analysis was used to determine cut-off points at the maximum Youden index. The kappa coefficient was used for measurement of agreement between clinical diagnosis and neurochemical testing. Fisher's z-transformation served for comparison of kappa-values. The statistical software package SPSS, version 10.0 was used for computations.

The DC group was significantly younger than AD (p=4.9x10⁻²) and VAD (p=7x10⁻³). Age and MMSE score did not significantly differ between VAD (mean ± SD) and AD (mean ± SD). The average time until freezing did not differ among the diagnostic groups. Hypertension was prevalent in 23%, 30% and 38% of DC, AD and VAD, respectively. Hypercholesterinemia was prevalent in 31%, 20% and 15% of DC, AD and VAD, respectively.

None of the investigated Aβ peptides, neither in absolute, nor percentage terms, correlated with age, MMSE or the occurrence of hypertension. Prevalent hypercholesterolemia correlated weakly with higher percentage Aβ1-38 levels (p=4.3x10⁻²). The time until freezing strongly correlated with lower overall Aβ peptide levels (p<0.01), but most interestingly not with any percentage Aβ peptide amount. The female gender was weakly correlated with higher levels of Aβ1-39, Aβ1-40 and Aβ1-42 (p<0.05).

DC and AD displayed no significant differences in any of the investigated peptides, neither in absolute, nor percentage terms. In contrast, absolute Aβ1-42 (p=1.2x10⁻²) and Aβ2-40 (p=8x10⁻³) were decreased in VAD as compared to a combined group of DC and AD. In percentage terms, there was a decrease of Aβ1-37 (p=8x10⁻³), Aβ1-38 (p=6.9x10⁻⁵), Aβ1-39 (p=8.7x10⁻⁴), Aβ1-42 (p=8.2x10⁻⁵) and Aβ2-40 (p=6.9x10⁻⁵), paralleled by elevated amounts of Aβ1-40 (p=4.6x10⁻³) in VAD as compared to a combined group of DC and AD.

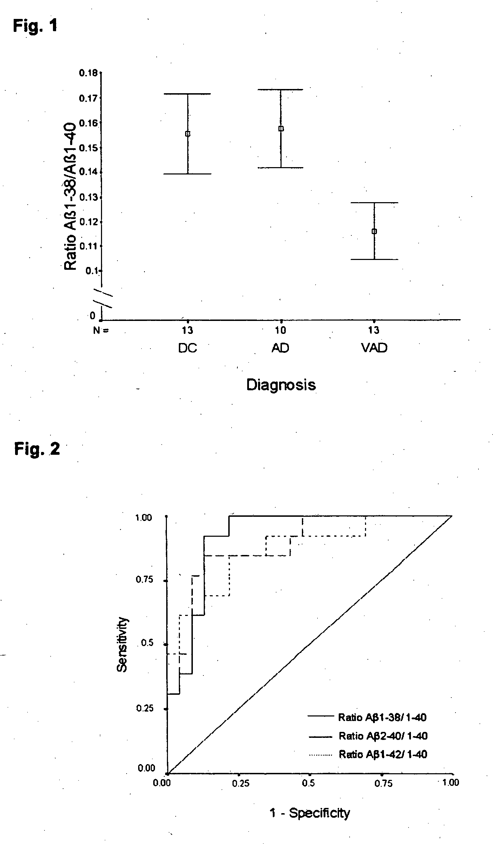

The most pronouncedly decreased peptides Aβ1-38, Aβ1-42 and Aβ2-40 were each combined with Aβ1-40 to the ratios Aβ1-38/ Aβ1-40, Aβ1-42/ Aβ1-40 and Aβ2-40/ Aβ1-40, respectively. **Fig. 1** visualizes the ratio Aβ1-38/ Aβ1-40 for all three diagnostic groups. ROC curve analysis as depicted in **Fig. 2** revealed areas under the curve (AUC) as follows: 0.92, 0.87 and 0.89 for Aβ1-38/ Aβ1-40, Aβ1-42/ Aβ1-40 and Aβ2-40/ Aβ1-40, respectively. Using a cut off point of 0.136, the ratio Aβ1-38/ Aβ1-40 exhibited a sensitivity of 92% for detection of VAD at a specificity of 87% in a combined group of AD and DC. The discriminative power of Aβ1-38/ Aβ1-40 between VAD and AD was even higher, yielding a sensitivity and specificity of 92% and 90%, respectively, at the same cut off point.

When VAD was tested for absolute Aβ1-40 in comparison to a combined group of AD and DC, the kappa coefficient gave a value of 0.386. In contrast, kappa coefficient gave a value of 0.767 for the ratio Aβ1-38/ Aβ1-40. After Ficher's z-transformation, the difference was significant to the level of 0.05.

### DISCUSSION

The presented results on detailed characterization of Aβ peptide species in plasma of VAD in comparison to AD and depressive controls are consistent with previous studies (Irizarry, 2004) in respect of Aβ1-40 and Aβ1-42. Most interestingly, a recent publication by Gurol et al. (2006) reported a correlation of increased Aβ1-40 to the load of white matter lesions in MCI, AD and CAA. This effect turned out to be independent of potential confounders, such as age, sex, hypertension, diabetes mellitus, APO-E status, homocysteine, folate and B12. Increased Aβ1-40 was interpreted as a potential cerebrovascular risk factor, and combined with our results, it may be a potential biomarker to distinguish pure AD from mixed AD/VAD dementia or pure VAD, respectively. The specific role of Aβ1-40 in VAD is underlined by the fact that this peptide comprises the major component of vascular amyloid plaques, but is less abundant in senile AD plaques (Gravina et al., 1995). It remains an open question whether the reduction of plasma Aβ peptides other than Aβ1-40 may also be closely related to the cerebrovascular amyloid pathology. However, the diagnostic power of this effect can be increased, when Aβ1-40 is assessed in relation to other Aβ peptides in plasma.

### EXAMPLE II

A total of 90 patients, whose CSF samples had been obtained for neurochemical analysis between 2000 and 2004, were divided into 3 diagnostic groups according to their clinical diagnosis: frontotemporal lobe degeneration (FTLD), Alzheimer's disease (AD), or non-demented disease controls (NDC). The CSF samples were analyzed by Aβ-SDS-PAGE/immunoblot for Aβ peptide patterns, and Aβ₁₋₄₂ and total tau protein were determined by ELISA.

The patients were selected from wards and the dementia outpatient clinic of the University of Göttingen (Göttingen, Germany). Five AD patients came from the dementia outpatient clinic of the University of Erlangen-Nuremberg (Erlangen, Germany). Both specialized centers followed a standardized protocol of sample handling. All neurochemical measurements and quantifications were performed in the laboratory of neurobiology of the University of Göttingen by two very experienced technical assistants blinded to clinical diagnosis. Diagnosis was established by a neurologist, a psychiatrist, and a neuropsychologist, all highly experienced in clinical differential diagnosis of dementias on the basis of thorough anamnesis, clinical examination, results of neuropsychological assessment, clinical records of the patients, and the best clinical judgement. All three investigators were blinded to the neurochemical outcome results. Investigations were carried out with the informed consent of all patients, or, for patients with severe dementia, their next of kin. If possible, neuropsychological assessment by the Mini-Mental-State-Evaluation (MMSE or Short Cognitive Performance Test (SKT) at minimum) was performed on patients suffering from cognitive impairments at the time of lumbar puncture.

The study was conducted under the guidelines of the Declaration of Helsinki (World Medical Organization 1996) and approved by the ethics committee of the Universities of Göttingen and Erlangen-Nuremberg.

### Patients with frontotemporal lobe degeneration:

The 30 patients (21 men and 9 women) of this group fulfilled the Diagnostic and Statistical Manual of Mental Disorders, Fourth Edition criteria (DSM IV) and the latest consensus criteria for FTLD (McKhann et al., 2001). The consensus criteria of Neary et al. (Neary et al., 1998) were applied to the clinical diagnosis of FTD (n = 24), primary progressive aphasia (PPA (n = 5) and semantic dementia (SD, n=1), respectively. Neuropsychological testing, including MMSE, clock drawing, the Consortium to Establish a Registry for Alzheimer's Disease neuropsychological battery (CERAD) and more detailed examination by experienced neurophysiologists was carried out on 24 patients. MMSE was available for 26 patients, the mean MMSE score was 20.7 ± 8.9 (mean ± SD (standard deviation)). Neuropsychological assessment was hindered in 4 patients due to severe linguistic or aphasic deficiencies. Age of this group was 61.6 ± 11.5 years (mean ± SD).

All enrolled patients underwent computerized tomography (CT) or magnetic resonance imaging (MRI) of the brain. Functional imaging using either 99mTc-hexamethylpropyleneamine oxime single photon emission computerized tomography (SPECT) or [18F]fluorodeoxyglucose PET investigation of the regional cerebral blood flow was available for 22 patients. Included patients exhibited frontal, frontotemporal, or anterior temporal focal atrophy or marked hypometabolism. Aβ 1-42 ELISA results were available for 22 patients, and tau ELISA for 25 patients.

### Patients with Alzheimer's disease:

All 30 patients (13 men and 17 women) of this group fulfilled the DSM IV criteria for AD and the NINCDS-ADRDA criteria for clinical diagnosis of probable AD (McKhann et al., 1984). The mean age of this group was 65.4 ± 7.3 years (mean ± SD). Neuropsychological testing, including MMSE, clock drawing, CERAD, and more detailed examination by experienced neurophysiologists was carried out on 29 patients. MMSE was not possible in one patient due to severe cognitive deficiency. The mean MMSE score was 19.3 ± 5.4 (mean ± SD) in this group. All patients underwent structural CT or MRI brain imaging. Included patients exhibited global cortical atrophy or temporal, parietotemporal, frontotemporal focal atrophy or marked hypometabolism of these regions was observed in 27 patients. There were no results for brain imaging of three patients. Results of Aβ₁₋₄₂ ELISA and tau ELISA were available for 26 patients.

### Non-demented disease controls (NDC):

This group consisted of 30 non-demented patients (10 men and 20 women), who underwent lumbar puncture for the differential diagnosis of dementia or other differential diagnostic reasons. Patients with persistent cognitive decline for more than six months, an MMSE score below 26, or clear focal atrophy in brain imaging (CT or MRI) were excluded from the study.

The mean age of this group was 61.5 ± 11.1 years (mean ± SD). The group included patients suffering from depression (n=16), panic attacks with vertigo (n=1), peripheral facial nerve palsy with borreliosis without central manifestation (n=1), facial hemispasm (n=1), antiphospholipidsyndrome (n=1), paraneoplastic syndrome (n=2), schizophrenia (n=1), polymorphic psychosis (n=1), mania (n=3), bipolar affective disorder (n=1), motor neuron disease without dementia (n=1), or insomnia (n=1). For assessing cognitive mental status all patients with cognitive complaints (n=22) were assessed by MMSE (Mini-Mental State Examination). The mean MMSE score was 28.5 ± 1.5 (mean ± SD). The cognitive impairment of all depressive patients improved after antidepressant medication. Brain imaging (CT or MRI) was available for 21 patients. Results of Aβ₁₋₄₂ ELISA and tau ELISA were available for 23 patients.

### Aβ-SDS-PAGE/immunoblot:

A volume of 10 µL CSF was boiled in sample buffer before analysis of Aβ peptides by SDS-PAGE. Aβ-SDS-PAGE/immunoblot was performed as described elsewhere (Wiltfang et al., 2002, Bibl et al., 2004).

CSF samples from each patient were run in triplicate, and each gel carried a four-fold dilution series of the synthetic Aβ peptides Aβ₁₋₃₇, Aβ₁₋₃₈, Aβ₁₋₃₉, Aβ₁₋₄₀, and Aβ₁₋₄₂. Synthetic peptides Aβ₁₋₃₈. Aβ₁₋₄₀, Aβ₁₋₄₂ were obtained from Bachem (Bubendorf, Switzerland), Aβ₁₋₃₇ and Aβ₁₋₃₉ were synthesized automatically according to Janek et al. (Janek et al., 2001). Synthetic Aβ peptide dilution series were created as described previously (Bibl et al., 2004). In detail, the stock solution of synthetic Aβ peptides (0.5 mg/mL) was diluted to 24 ng/mL (Aβ₁₋₄₀), 12 ng/mL (Aβ₁₋₃₈ and Aβ₁₋₄₂), and 6 ng/mL (Aβ₁₋₃₇ and Aβ₁₋₃₉). The peptides were mixed to create the first dilution step of synthetic Aβ peptide dilution series and the mixture was diluted 1:2 three more times.

The amino-terminal-selective mouse monoclonal antibody 1 E8 (Schering, Berlin, Germany) was applied overnight at 4°C for detection of the antigen (N-terminus with the first two aminoacids of Aβ peptides 1-X). Further 1-hour incubations with a biotinylated anti-mouse polyclonal antibody (Vector Laboratories, Burlingame, California, USA) and horseradish peroxidase-coupled streptavidin (Amersham Pharmacia Biotech, Hertfordshire, UK) were performed. Washing steps were performed between each incubation. Visualization of the resulting bands was by enhanced chemiluminescence (ECL), A charge-coupled device (CCD). camera was used to quantify the bands by comparison with the standard Aβ peptide dilution series.

The detection sensitivity of the 1 E8 in this optimized immunoblot procedure was 0.6 pg (Aβ₁₋₃₈, Aβ₁₋₄₀) and 1 pg (Aβ₁₋₃₇. Aβ₁₋₃₉, Aβ₁₋₄₂), respectively (Bibl et al., 2004). The inter- and intra-assay coefficients of variation were below 10% for 80 and 20 pg of the synthetic Aβ peptides (Wiltfang et al., 2002; Bibl et al., 2004).

### Electrochemiluminescence detection of Aβ₁₋₃₈

The novel electrochemiluminescence detection technology (MSD) was applied to determine CSF Aβ₁₋₃₈ levels independently of the Aβ-SDS-PAGE/ immunoblot. It was conducted according to the manufacturer's recommendations (Meso Scale Discovery, Gaithersburg, Maryland, USA). In brief, Multi-Spot 4, 96-well plates (Meso Scale Discovery, Gaithersburg, Maryland, USA), precoated with the N-terminal-specific anti-Aβ antibody 6E10 were blocked with solution A for one hour. The plates were then incubated for one hour each with peptide dilution series or 100 µL CSF samples, followed by c-terminal SULFO-TAG Aβ 1-38 detection antibody (Meso Scale Discovery, Gaithersburg, Maryland, USA), and read buffer. Washing with 1xTris buffer was performed between all incubation steps. The emitted light was read at a wavelength of - 620nm.

### Tau protein ELISA and Aβ 1-42 ELISA:

The commercially available assays INNOTEST^{®} hTAU Antigen and INNOTEST^{®} β-Amyloid ₍₁₋₄₂₎, Innogenetics (Gent, Belgium) were used for the quantification of tau protein and Aβ 1-42 levels, respectively, in CSF. The assays were performed according to previously published standard methods (Hulstaert et al., 1999).

The inter- and intra-assay variability of the Aβ1-42-ELISA was below 10%, the detection sensitivity for Aβ1-42 was 50 pg/mL (Hulstaert et al., 1999).

### Statistical analysis:

Aβ peptide levels were expressed as absolute values (ng/mL). The data on peptide levels was obtained from individual blots of each patient sample. Patient groups were characterized by mean and standard deviation (SD).

The Mann-Whitney U-test was applied to evaluate significant group differences. The Wilcoxon signed ranks test was used to calculate significant differences of two related samples. Multiple comparisons were not performed.

The two-sided level of significance was taken as p < 0.05. A p-value of less than 0.01 was considered as highly significant.

Receiver operating characteristic (ROC) curve analysis was used to determine cutoff points. Additionally, the optimal cutoff level for dichotomizing values was selected as the situation maximizing the Youden index. Only the best-discriminating cutoff values alone or in combination are presented.

Computations were performed using the statistical software package SPSS, version 10.0.

### RESULTS:

The mean age of the diagnostic groups did not differ significantly (p> 0.05).

The mean MMSE score between FTLD and AD did not differ significantly (p> 0.05) but was significantly higher for the NDC group (p= 1.5x10⁻⁷ and 1.5x10⁻⁶, respectively).

The gender distribution was imbalanced among the diagnostic groups. However, no correlation of any of the investigated Aβ peptides with gender was found.

### Aβ- peptide patterns in the Aβ-SDS-PAGE/immunoblot:

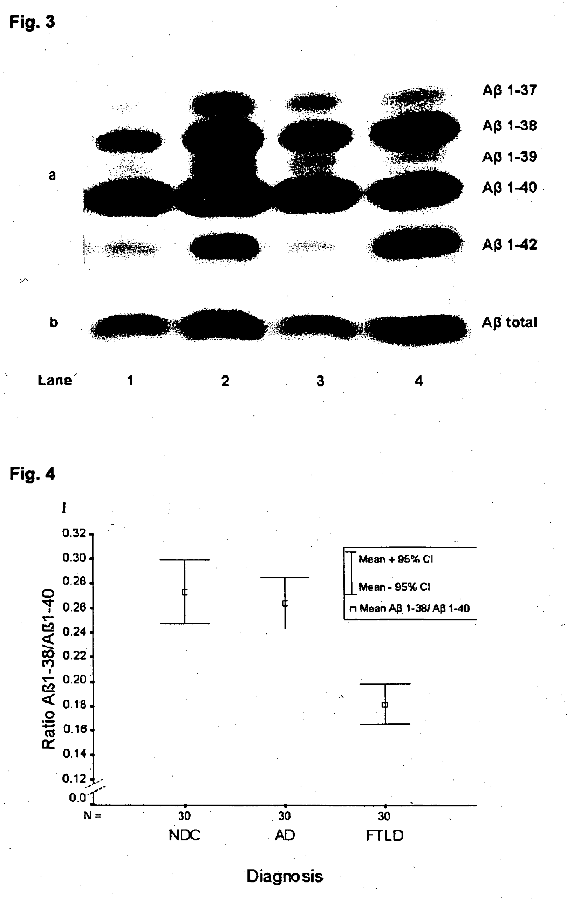

In the presence of urea, the Aβ-SDS-PAGE/immunoblot allowed the electrophoretic separation and subsequent analysis of Aβ1-37, Aβ1-38, Aβ1-39 and Aβ1-40 in addition to Aβ1-42. All Aβ peptides migrate as a single band, if urea is absent in otherwise unchanged separation gels **(****Fig. 3****).**

The total amount of Aβ peptides did not differ significantly among the diagnostic groups (p>0.05). Both dementia groups, FTLD and AD, displayed decreased absolute levels of Aβ1-42 relative to the NDC group as measured by the Aβ-SDS-PAGE/immunoblot. However, this decrease was much more pronounced in AD (p= 3.3x 10⁻⁴, for FTLD-group,and p= 8.5x10⁻¹⁰, for AD-group respectively). In contrast, the Ct-truncated Aβ peptides Aβ1-37 and Aβ1-38 were selectively reduced in FTLD as compared to NDC (p=2.7x10⁻⁴ and p= 4.2x10⁻⁵) and AD (p=2.5x10⁻³ and p= 1.2x10⁻³), respectively. Aβ1-40 was slightly elevated in FTLD and AD, whereas Aβ1-39 was found to be increased only in AD and decreased in FTLD. None of these alterations were significant. In contrast, the percentage concentration of Aβ1-40 relative to the sum of all measured Aβ peptides (Aβ1-40%) was significantly increased in FTLD (p= 2.2x10⁻⁷). The absolute and percentage abundances of Aβ peptides of each diagnostic group are summarized in Table 6.

Decreased levels of Aβ1-38 in FTLD allowed a distinction from AD with a sensitivity of 77% and specificity of 67%. The less pronounced decrease of Aβ1-42 in FTLD enabled discrimination from AD with a sensitivity of 87% and a specificity of 70%. Of all peptides investigated, these two peptides displayed the highest accuracy as a single marker to differentiate between FTLD and AD. This prompted us to investigate the diagnostic accuracy of a combination of both peptides. the ratio of Aβ1-42 to Aβ1-38 exhibited a specificity of 97% and sensitivity of 93% for differentiation of FTLD from AD.

Despite similar alterations of the two peptides in FTLD as compared to NDC, the Aβ1-42/ Aβ1-38 ratio showed no reasonable accuracy in the differentiation of FTLD from NDC or in a combined group of AD and NDC.

With a sensitivity of 77% and a specificity of 73%, the reduction of Aβ1-38 in FTLD achieved the maximum diagnostic accuracy among the absolute values of each single Aβ peptide to differentiate FTLD from NDC.

Taken together with the slightly elevated Aβ1-40 levels in FTLD, the ratio of Aβ1-38 to Aβ1-40 improved the discrimination of FTLD and NDC to a sensitivity of 87 % and a specificity of 90%. Additionally, this ratio provided a sensitivity of 87% and a specificity of 87% for differentiation of FTLD from AD.

Moreover, the ratio of Aβ1-38 to Aβ1-40 achieved the best discrimination of FTLD among both NDC and AD, yielding a sensitivity and specificity of 87% and 88%, respectively.

The cutoff points, sensitivities, and specificities for each differential diagnostic testing method are summarized in Table 8.

### Determination of tau protein and Aβ1-42 by ELISA

Tau protein was significantly elevated in the AD group (p=3.6x10⁻⁷), but only marginally in FTLD (p>0.05), as compared to NDC. This assay reached a sensitivity of 91% and a specificity of 81% for the discrimination of FTLD and AD. The sensitivity and specificity of FTLD detection among NDC was 50% and 61%, respectively.

The FTLD and AD groups displayed decreased absolute levels of Aβ1-42 compared to the NDC group as measured by ELISA (p= 3x10⁻³and 9.7x10⁻⁸, respectively). Absolute Aβ1-42 levels discriminated FTLD and AD with a sensitivity of 91% and a specificity of 89%. FTLD could be distinguished from NDC with a sensitivity of 68% and a specificity of 70%.

By combining Aβ1-42 and tau protein levels in a ratio of Aβ1-42/tau, the diagnostic power of the test for FTLD detection among AD was increased to a sensitivity of 86 % and a specificity of 100%. The sensitivity and specificity for detection of FTLD among NDC patients was 81% and 61%, respectively. The detection of FTLD among both NDC and AD yielded a sensitivity of 100% and specificity of 45%.

The absolute values for Aβ1-42 and tau as measured by ELISA, and the cutoff points, sensitivities, specificities, and maximum Youden index for each differential diagnostic testing method, are summarized in Tables 6 and 8, respectively.

### Aβ 1-42 levels measured by different methods

Aβ1-42 was measured by both the commercially available ELISA and Aβ1-SDS-PAGE/immunoblot. The comparison of values revealed higher levels of Aβ 1-42 when determined by the Aβ-SDS-PAGE/immunoblot. In a total of 71 patient samples, the difference was highly significant (p= 2.8x10⁻¹²). The degree of difference per diagnostic group were found to be as follows: p= 4.6x10⁻⁶ for the NDC group (n=23), 2.1x 10⁻⁵ for AD (n=26), and 1.2x10⁻⁴ for FTLD (n=22).

### EXAMPLE III

303 consecutive CSF patient samples obtained between 1999 and 2004 were prospectively investigated. Seventy-four of these patients had been investigated in previous studies under another objective and the results have been published. These patients were grouped by diagnosis as follows: NDC (n=15), AD (n=21), DLB (n=20), and OD (n=18).

CSF from patients diagnosed as having AD, FTD, and other dementias, as well as three patients with depression, were obtained from the memory clinic of the University of Goettingen, and samples from other non-demented disease controls were obtained from inpatients. Samples from 7 AD patients came from the dementia outpatient clinic of the University of Erlangen-Nuremberg. The CSF samples of patients hospitalized with Parkinson's disease (PD), Parkinson's disease dementia (PDD), and DLB were collected in the Paracelsus-Elena Klinik, Kassel, Germany, which specializes in the diagnosis and treatment of movement disorders.

Diagnoses were rendered by a psychiatrist, a neurologist and a neuro-psychologist, who were all very experienced in clinical differential diagnosis of dementias, on the basis of thorough anamnesis, clinical examination, results of a neuropsychological assessment, clinical records, and best clinical judgement. All three investigators were blinded to the results of the neurochemical outcome measurements. Investigations were carried out with the informed consent of patients or their authorized caregiver. If possible, neuropsychological assessment (MMSE at minimum) was performed on patients suffering from cognitive impairments at the time of lumbar puncture. The study was conducted under the guidelines of the Declaration of Helsinki and approved by the ethics committees of the Universities of Goettingen, Erlangen-Nuremberg and Hessen.

### Non-demented disease controls (NDC):

Patients with a history of persistent cognitive decline for more than six months, MMSE score below 26, or clear focal atrophy in brain imaging were excluded.

The NDC group consisted of three subgroups:

### Peripheral neurological diseases without organic brain affection (PND):

Twenty patients (13 men and 7 women) underwent lumbar puncture for exclusion of central nervous affection in cases of polyneuropathy (n=11), peripheral facial nerve palsy (n=3), benign paroxysmal positioning vertigo (n=2), intervertebral disc herniation (n=1), facial hemispasm (n=1), autosomal dominant hereditary spastic spinal palsy (n=1), and Lyme disease without central nervous affection (n=1). The mean age of this subgroup was 63.0 ± 10.3 years (mean ± SD).

### Neurodegenerative diseases without dementia (ND):

Twenty-five patients (14 men and 11 women) underwent lumbar puncture to exclude chronic inflammatory central nervous disease in cases of genetically reconfirmed Huntington's disease (n=10), Parkinson's disease (n=7), multisystem atrophy (n=5), spinocerebellar ataxia (n=2), and amyotrophic lateral sclerosis (n=1). The MMSE score in patients with cognitive complaints (n=9) was 28.2 ± 1.6 (mean ± SD). None of these patients displayed clinical features of dementia. The mean age of this subgroup was 62.3 ± 9.5 years (mean ± SD).

### Depressive cognitive complainers (DCC):

Twenty-six depressive patients (8 men and 18 women) underwent lumbar puncture for differential diagnosis of cognitive complaints during the course of disease. The diagnosis of depression was made according to the DSM IV criteria and cognitive impairment was assessed by MMSE at minimum. The mean MMSE score was 28.6 ± 1.4 (mean ± SD). The mean age of this subgroup was 62.9 ± 10.3 years (mean ± SD).

### Patients with Alzheimer's disease:

Seventy-one patients (29 men and 42 women) fulfilled DSM IV criteria and NINCDS-ADRDA criteria for clinical diagnosis of AD⁹. Structural (CT or MRI) or functional (SPECT or PET) brain imaging, respectively, displayed global cortical atrophy; temporal, parietotemporal, or frontotemporal focal atrophy; or marked hypometabolism of these regions.

### Patients with dementia with Lewy bodies (DLB):

Thirty-two patients (19 men and 13 women) fulfilled the DSM IV criteria for dementia and the McKeith criteria for clinical diagnosis of DLB¹⁰.

Patients presented with at least two core features according to the criteria and with parkinsonism for less than one year before onset of dementia. Enrolled patients were hospitalized for several days to evaluate fluctuating cognition, extrapyramidal symptoms, and visual hallucinations.

### Patients with frontotemporal dementias (FTD):

All 36 patients (22 men and 14 women) of this group fulfilled the DSM IV and the consensus criteria for FTD. Detailed neuropsychological testing additional to MMSE, including clock drawing and CERAD, was carried out on 23 patients. Neuropsychological assessment was hindered in 5 patients by severe linguistic or cognitive deficiencies. Structural (CT or MRI) or functional (SPECT or PET) brain imaging revealed frontal or frontotemporal focal atrophy or marked hypometabolism.

### Patients with other dementias (OD):

Ninety-three patients (57 men and 36 women) fulfilled the DSM IV criteria for dementia.

Patients with primary progressive aphasia (PPA) (n=10) fulfilled the consensus criteria of Neary et al. Structural or functional (SPECT or PET) brain imaging revealed left anterior temporal focal atrophy or marked hypometabolism.

The diagnosis of vascular dementia (VAD) was made in 27 patients according to NINDS-AIREN criteria. All patients exhibited signs of relevant vascular disease in structural brain imaging (CT or MRI).

Parkinson's disease dementia (PDD) was diagnosed in 21 patients according to the UK Parkinson's Disease Society Brain Bank clinical diagnostic criteria for idiopathic Parkinson's disease and the consensus criteria. All patients presented with parkinsonism at least one year before onset of dementia.

Normal pressure hydrocephalus according to the proposed criteria of Ishikawa was diagnosed in nine patients. All these patients exhibited at least two symptoms of the typical triad and improved after spinal tap.

Six patients fulfilled the criteria of probable progressive supranuclear palsy according to established NINDS-SPSP criteria.

Six patients were diagnosed as having corticobasal degeneration according to the established criteria.

Seven patients suffering from sporadic Creutzfeld-Jakob's disease (CJD) were evaluated according to established criteria at the national surveillance unit for transmissible spongiform encephalopathies in Goettingen, Germany.

Seven patients with Korsakow's syndrome were evaluated according to the criteria of Oslin and colleagues.

The mean age and MMSE score of each patient group are given in Table 7.

### TEST METHODS

The pre-analytical handling of CSF samples followed a standardized protocol according to previously published data.

### Aβ-SDS-PAGE/immunoblot

For analysis of Aβ peptides, 10 µL of CSF was boiled in SDS-PAGE sample buffer, separated on urea-containing *N*,*N_*-bis-(2-hydroxyethyl)-glycine/bis-Tris/Tris/sulfate SDS-PAGE gels and Aβ-SDS-PAGE/immunoblot was conducted as published elsewhere.

CSF samples of each individual patient were run as triplicates. Bands were quantified from individual blots of each patient relative to a four-point dilution series of synthetic Aβ peptides using a charge-coupled device camera. The detection sensitivity was 0.6 pg (Aβ₁₋₃₈, Aβ₁₋₄₀) and 1 pg (Aβ₁₋₃₇, Aβ₁₋₃₉, Aβ₁₋₄₂), respectively. The inter- and intra-assay coefficients of variation for 20 to 80 pg of synthetic Aβ peptides were below 10%.

All neurochemical measurements and quantifications were performed in the neurobiology laboratory of the University of Goettingen between 2003 and 2006 by two very experienced technical assistants blinded to clinical diagnosis.

### Electrochemiluminescence detection of Aβ1-38

The novel electrochemiluminescence detection technology (MSD) was applied to determine CSF Aβ₁₋₃₈ levels independently of the Aβ-SDS-PAGE/ immunoblot. It was conducted according to the manufacturer's recommendations (Meso Scale Discovery). In brief, Multi-Spot 4, 96 well plates, precoated with the N-terminal-specific anti-Aβ antibody 6E10 were blocked with solution A for one hour. The plate was then incubated with peptide dilution series or 100 µL CSF sample, followed by C-terminal SULFO-TAG Aβ₁₋₃₈ detection antibody and Read Buffer, each for one hour. Washing with 1xTris buffer was performed between incubation steps. The measurement of emitted light was performed at - 620 nm.

### STATISTICAL ANALYSIS:

Patient groups were characterized by mean and standard deviation. Aβ peptide values are given in absolute (ng/mL) and percentage values relative to the sum of all investigated Aβ peptides (Aβ₁₋ₓ%). The Mann-Whitney U-test was employed to determine significant differences of diagnostic groups (unpaired samples). Comparisons of multiple groups (age, MMSE) were evaluated by the Kruskal-Wallis test. Correlations of measured values were estimated by Spearman's Rho. The two-sided level of significance was taken as p<0.05. The global diagnostic accuracies were assessed by the area under the curve (AUC) of receiver operating characteristic curve (ROC). Cut-off points were determined at the maximum Youden index, providing a sensitivity of ≥85%. The statistical software packages SPSS, version 10.0 and SAS, version 8.2 served for computations.

### TEST RESULTS

The mean age of FTD and NDC was significantly younger than all other patient groups. The mean MMSE score did not significantly differ between the dementia groups. The Aβ-SDS-PAGE/immunoblot revealed a regular abundant pattern of six peptides: Aβ₁₋₄₀, Aβ₁₋₃₈, Aβ₁₋₄₂, Aβ₁₋₃₉, Aβ₁₋₃₇ and Aβ₁₋₄₀^{ox}.

No correlation of the investigated Aβ peptides with age was found in any of the diagnostic groups.

### Neurochemical phenotype in NDC

There was no significant difference among PND and ND. In contrast, DCC exhibited higher absolute levels of Aβ₁₋₃₇ (p=1.3x10⁻³), Aβ₁₋₃₈ (p=4.9x10⁻²) and Aβ₁₋₄₂ (p=4.0x10⁻³) than a combined group of PND and ND. In percentage terms, Aβ₁₋₃₇% (p=2.9x10⁻⁴) and Aβ₁₋₄₂% (p=1.5x10⁻³) were increased, paralleled by diminished Aβ₁₋₄₀% (p=3.3x10⁻³) and Aβ₁₋₄₀^{ox}% (p=1.4x10⁻²) values in DCC.

### Neurochemical phenotype in AD

### AD versus NDC:

AD presented with clearly decreased Aβ₁₋₄₂ levels in absolute (p=7.4x10⁻¹⁹) and percentage terms (p=3.8x10⁻²⁴), whereas Aβ₁₋₄₀^{ox} levels were increased in absolute (p=1.1x10⁻²) and percentage terms (p=1.8x10⁻⁵). Additionally, there was a percentage increase of all peptides with C-terminal truncation that were shorter than Aβ₁₋₄₂, which failed the level of significance for Aβ₁₋₃₇% and Aβ₁₋₃₈%. The elevation of Aβ₁₋₃₈% was highly significant compared with the ND group (p=4.6x10⁻³).

### AD versus other dementias

A specific decrease of Aβ₁₋₄₂ was evident in AD, whereas other dementias with low Aβ₁₋₄₂ levels displayed an overall decrease of all Aβ peptides. Correspondingly, Aβ₁₋₄₂was reduced in absolute (p=4.1x10⁻⁷) and percentage (p=7.6x10⁻²²) terms. In contrast, absolute levels of Aβ₁₋₃₇ (p=2.5x10⁻²), Aβ (p=5.0x10⁻⁵), Aβ (p=2.4x10⁻³) and Aβ (p=6.3x10⁻³) were elevated. In percentage terms, the aforementioned alterations were only present for Aβ₁₋₃₈%, Aβ ₁₋₃₉% and Aβ₁₋₄₂%, respectively.

### Neurochemical phenotype in FTD

### FTD versus NDC

FTD showed lower levels of Aβ₁₋₃₇ (p=2.3x10⁻⁴), Aβ₁₋₃₈ (p=9.6x10⁻⁷) and Aβ₁₋₄₂ (p=6x10⁻⁵). In percentage terms, there was an additional increase of Aβ% values (p= 7x10⁻¹⁰).

### FTD versus other dementias:

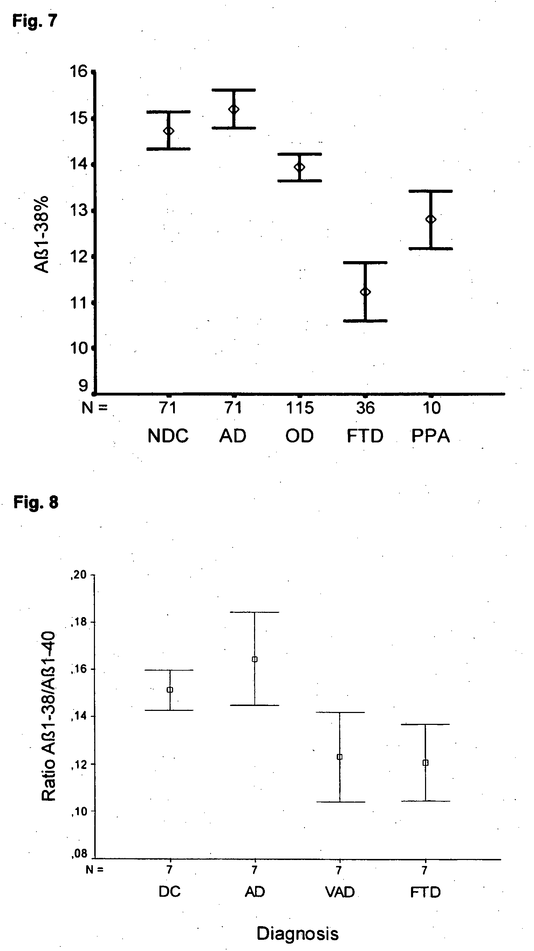

FTD presented lower Aβ (p=2.1x10⁻²) and Aβ^{ox} (p=5.0x10⁻³) levels, whereas Aβ levels were elevated (p= 3.8x10⁻³). In percentage terms, there were drops in Aβ₁₋₃₇% (p=2.5x10⁻⁴), Aβ₁₋₃₈% (p=3.5x10⁻¹⁵), Aβ₁₋₃₉% (p=10x10⁻²) and Aβ₁₋₄₀^{ox}% (p=2.6x10⁻⁴), paralleled by elevated levels for Aβ₁₋₄₀% (p=1.9x10⁻⁵) and Aβ₁₋₄₂% (p=6.2x10⁻⁴). Compared to PPA, there were decreased absolute Aβ₁₋₃₇ (p=1.1x10⁻²), Aβ₁₋₃₈ (p=1x10⁻²) and Aβ₁₋₃₉ (p=1.8x10⁻²) levels in FTD. The levels of significance were as follows (for Aß1-38 % also see **Fig.** 7):

| | Aß1-37 (ng/ml) | Aß1-38 (ng/ml) | Aß1-37 % | Aß1-38 % |
|---|---|---|---|---|
| p-value | .043 | .003 | .011 | .010 |

Additionally, Aβ₁₋₄₀% was elevated (p=1.8x10⁻³) in FTD.

### Aβ₁₋₃₈ in the Aβ-SDS-PAGE/immunoblot and electrochemiluminescence detection (MSD)

A total of 150 patients was reevaluated using novel electrochemiluminescence detection technology (MSD). Diagnoses were NDC (n=37), AD (n=31), DLB (n=2), OD (n=47), and FTD (n=33). Observed absolute levels of peptide concentration were considerably lower using MSD compared with the Aβ-SDS-PAGE/ immunoblot (see Table 7). Conversely, there was a strong correlation of values between the two independent methods of measurement (Spearman's Rho= 0.45, p=7.9x10⁻⁹ **(see** **Fig. 4****).**

### ESTIMATES

### Neurochemically supported differential diagnosis

### Diagnosis of AD

The striking drop of Aβ₁₋₄₂% enabled contrasts beyond 85% for discrimination of AD among the total of all investigated patients. A sensitivity of 85% gave a specificity of 81 % for exclusion of all non-Alzheimer dementias. Due to decreased values of Aβ₁₋₃₈ and a less marked drop of Aβ₁₋₄₂ in dementias other than AD, the ratio of Aβ₁₋₃₈ to Aβ₁₋₄₂ (Aβ₁₋₄₂/ Aβ ₁₋₃₈) improved the test slightly to contrasts of 85% or beyond for all investigated differential diagnostic questions. Otherwise, the sole absolute values of Aβ₁₋₄₂ yielded a specificity of 50% for exclusion of non-Alzheimer dementias, when the sensitivity for AD detection was set to a minimum of 85%.

### Diagnosis of FTD

The pronounced percentage reduction of Aβ₁₋₃₈% in FTD exhibited satisfactory accuracies of above 85% for discrimination among all other dementias and NDC. The combination of decreased Aβ₁₋₃₈ and elevated Aβ₁₋₄₀ (Aβ₁₋₃₈/Aβ₁₋₄₀) levels just failed to fulfill the requirements for differentiation of FTD and other dementias, but still exhibited contrasts of beyond 85% for differentiation of FTD from NDC. The loss of accuracy of the Aβ₁₋₃₈/Aβ₁₋₄₀ ratio as compared to Aβ₁₋₃₈% was mainly due to elevated Aβ₁₋₄₀ levels in individual AD, DLB, and PPA patients.

### EXAMPLE IV

This example aimed at obtaining detailed knowledge about the plasma Aβ peptide patterns in VAD and FTD.

28 plasma samples obtained between 2000 and 2005 were investigated. Plasma and CSF samples of AD, VAD, FTD and patients with depression came from the memory clinic of the Department of Psychiatry, University of Goettingen or from hospitalized patients. All patients, except patients with FTD and one with vascular dementia have been investigated in a previous study and the results have been published (Bibl et al., 2007a)

A psychiatrist and a neurologist, both very experienced in clinical differential diagnosis of dementias, rendered diagnoses based on thorough anamnesis, clinical examination, results of neuropsychological assessment, clinical records and best clinical judgement. These investigators were blinded to the neurochemical outcome measures. Investigations were carried out with the informed consent of patients or their authorized caregiver. If possible, neuropsychological assessment (MMSE at minimum) was performed on patients suffering from cognitive impairments at the time of lumbar puncture. The study was conducted under the guidelines of the Declaration of Helsinki (World Medical Organization 1996) and approved by the ethics committee of the University of Goettingen, Erlangen-Nuremberg and Hessen.

### Non-demented depressive controls (DC):

The 7 patients with depression (2 men and 5 women) underwent lumbar puncture for differential diagnosis of cognitive complaints during the course of disease. The diagnosis of depression was made according to the criteria of DSM IV and cognitive impairment was assessed by MMSE at minimum. Patients with history of persistent cognitive decline for more than six months, MMSE score below 26, clear focal atrophy or with signs of relevant vascular disease in brain imaging were excluded.

### Patients with Alzheimer's disease (AD):

These 7 patients (2 men and 5 women) fulfilled DSM IV criteria and NINCDS-ADRDA criteria for clinical diagnosis of AD (McKhann et al. 1984). AD patients displayed no signs of relevant cerebrovascular disease in either CT or MRI.

### Patients with vascular dementias (VAD) and AD with CVD:

We investigated 7 patients with VAD (3 men and 4 women) according to DSM IV and the NINDS-AIREN criteria (Roman et al. 1993). Patients were further characterized by Hachinski's ischemic scale in its modified version according to Fischer et al. (1991) and patients with a score less than six were excluded. All included patients exhibited typical signs of relevant cerebrovascular disease in neuroimaging (computerized tomography, magnetic resonance imaging). Table 10 shows the patients' characteristics of each patient group.

### Patients with Frontotemporal dementia (FTD):

All 7 patients (5 men and 2 women) of this group fulfilled the DSM IV and the consensus criteria for FTD (Neary et al. 1998). Structural (CT or MRI) or functional (SPECT or PET) brain imaging revealed frontal or frontotemporal focal atrophy or marked hypometabolism.

### TEST METHODS

CSF and plasma were drawn from patients by lumbar and venous puncture, respectively, on the same day. The preanalytical handling of samples followed a standardized protocol according to previously published data, except that the time span until freezing of plasma ranged up to 12 hours (Lewczuk et al. 2004).

Neither an intermediate temperature stage, nor shock freezing was performed. There was no additional freeze and thaw cycle before analysis. CSF was sampled in polypropylene vials and centrifuged (1000g, 10 min, and 4°C). Aliquots of 200 µl CSF were kept at +4°C and analyzed within 48 hours.

Two experienced technical assistants blinded to the clinical diagnosis performed all neurochemical measurements and quantifications at the laboratory of the Department of Psychiatry, University of Goettingen.

### Immunoprecipitation and Aβ-SDS-PAGE/immunoblot of plasma

For immunoprecipitation, magnetic sheep anti-mouse IgG Dynabeads M-280 (Dynal, Hamburg, Germany) were incubated overnight at +4°C with the monoclonal Aβ amino-terminally-selective antibody, 1E8 (provided by Schering, Berlin, Germany) according to the manufacturer's protocol. Five hundred µL of plasma were added to 200 µL of five-fold concentrated RIPA detergent buffer (Wiltfang et al. 2002), 25µL of the activated magnetic Dynabeads (1 µg mAb 1E8/1.68 x 10⁷ beads) and 300 µL of H₂0. Samples were then incubated overnight at +4°C under rotation, following washing of the beads four times with PBS/0.1% of bovine serum albumin and once with 10 mM Tris/HCl, pH 7.4. For Aβ-SDS-PAGE/immunolot, bound Aβ peptides were eluted by heating the samples to 95°C for 5 min with 25µL of SDS-PAGE sample buffer (Wiltfang et al. 2002).

For the separation of Aβ peptides, the urea version of the Bicine-/bis-TrislTris/sulfate SDS-PAGE was used as described by Wiltfang et al. (2002). Briefly, immunoprecipitated plasma (5 µL of immunoprecipitate) in SDS-PAGE sample buffer was applied to a gel slot. After running the gels at a constant current of 24mA per gel at room temperature for 1 h, semidry Western blotting was performed according to the protocol of Wiltfang et al (2002). For immunological detection of Aβ peptides on PVDF-membranes, the amino-terminally-selective mouse monoclonal antibody 1E8 (provided by Schering, Berlin, Germany; stock: 0.25 mg/ mL) was diluted 4000-fold and applied overnight at 4°C. Membranes were further incubated with a biotinylated anti-mouse polyclonal antibody (Vector Laboratories, Burlingame, CA, USA) and horseradish peroxidase coupled streptavidin (Amersham Pharmacia Biotech, Buckinghamshire, England) for 1 h each. Washing steps were performed in between and chemiluminescent visualization was done by ECL-Plus solution (Amersham Pharmacia).

Plasma samples and peptide standards were run on the same gel as triplicates at minimum. Each gel carried a five point dilution series of synthetic Aβ peptides Aβ1-37, Aβ1-38, Aβ1-39, Aβ1-40 and Aβ1-42. Aβ2-40 was only applied in the two lower concentrations, because bands of Aβ2-40 and Aβ1-42 tended to diffuse into one single band when higher concentrations were applied. Thus, the three higher concentrations of dilution series of Aβ1-42 were used to complement the standard row of Aβ2-40. Synthetic peptides Aβ1-38, Aβ1-40, Aβ1-42 and Aβ2-40 were obtained from Bachem (Bubendorf, Switzerland), Aβ1-37 and Aβ1-39 were synthesized automatically according to Janek et al. (Janek K et al 2001). Standard preparations of synthetic Aβ peptide mixture were created as described previously (Bibl M et al. 2004) and bands were quantified from individual blots of each patient relative to this dilution series using a charge coupled device camera (CCD-camera) and Quantity-ONE software (FluorSMax Multilmager, Bio-Rad)

The detection sensitivity of the Aβ-SDS-PAGE/immunoblot was 0.6-1 pg (Wiltfang et al. 2002, Bibl et al. 2004). Coefficients of variation of the Aβ peptides' concentrations ranged between 14.0 and 18.5% (Lewczuk et al. 2004).

### STATISTICAL ANALYSIS

Patient groups were characterized by mean and standard deviation. CSF peptide levels are given in absolute values (pg/ml). Plasma peptide values are given in absolute (pg/ml) or percentage (amount of each peptide relative to the sum of all single investigated Aβ peptides) values, respectively. Significant group differences were evaluated with the Mann-Whitney U-test. Comparisons of multiple groups were evaluated by the Kruskal-Wallis test. Spearman's rho was used for correlation analysis. We used the biserial correlation coefficient to determine the relationship between clinical diagnosis and neurochemical testing. Fisher's z-transformation served for comparison of biserial correlation coefficients. The two-sided level of significance was taken as p<0.05. The global diagnostic accuracies were assessed by the area under the curve (AUC) of receiver operating characteristic curve (ROC). Cut-off points ware determined at the maximum Youden index, providing a sensitivity of ≥85%. The statistical software package SPSS, version 10.0 was used for computations.

### RESULTS

### Group differences

Age did not significantly differ among the diagnostic groups. The average time until freezing and MMSE score, respectively, did not significantly differ between AD, FTD or VAD (mean ± SD).

### Plasma Aβ peptides in AD

Plasma Aβ peptides in DC and AD did not significantly differ.

### Plasma Aβ peptides in VAD

The comparison of VAD with a combined group of DC and AD revealed higher absolute values of Aβ1-40 (p=5.6x10⁻³) and Aβ1-39 (p=3.1x10⁻³) in VAD. Consequently, the sum of all investigated Aβ was higher in VAD (p=4.2x10⁻³). In percentage terms, this was expressed by a decrease of Aβ1-38 (p=1x10⁻²), Aβ1-42 (p=1.6x10⁻³) and Aβ2-40 (p=4.6x10⁻²), paralleled by elevated amounts of Aβ1-40 (p=1.6x10⁻²) in VAD.

### Plasma Aβ peptides in FTD

The comparison of FTD with a combined group of DC and AD revealed no significant difference in any of the investigated Aβ peptides in absolute terms. Conversely, the percentage amounts of Aβ1-37 (p=5.6x10⁻³) and Aβ1-38 (p=1.1x10⁻³) were significantly decreased in FTD, whereas percentage levels of Aβ1-40 were increased in FTD (p=4.6x10⁻²).

### Plasma Aβ peptides in FTD versus VAD

Interestingly, FTD and VAD displayed similarities in elevated percentage levels of Aβ1-40 and decreased percentage levels of Aβ1-38 as compared to DC and AD. Conversely, percentage levels of Aβ₁₋₄₂ and Aβ2-40 were diminished only in VAD, but not in FTD.

Direct comparison of FTD and VAD revealed pronouncedly decreased levels of absolute Aβ1-37 (p=3.8x10⁻²) and Aβ1-38 (p=3.8x10⁻²) levels in FTD. Otherwise, percentage levels of Aβ1-42 levels lower in VAD (p=7.0x10⁻³)

### TEST ACCURACIES

### Diagnosis of VAD

The positive diagnosis of VAD among DC and AD could be most effectively supported by elevated levels of Aβ1-40 accompanied by decreased levels of Aβ1-38 and Aβ1-42, respectively. Both ratios Aβ1-38/Aβ1-40 and Aβ1-40/Aβ1-42 yielded an AUC of 0.92.

### Diagnosis of FTD

The positive diagnosis of FTD among DC and AD could be most effectively supported by elevated levels of Aβ1-40 accompanied by decreased levels of Aβ1-38 and Aβ1-37, respectively. The ratios Aβ1-38/Aβ1-40 and Aβ1-37/Aβ1-40 yielded an AUC of 0.95 and 0.90, respectively.

### Differential diagnosis of FTD and VAD

The positive diagnosis of FTD among VAD could be most effectively supported by higher levels of Aβ₁₋₄₂ accompanied by decreased levels of Aβ1-38 and Aβ1-37, respectively. The ratios Aβ1-38/Aβ1-42 and Aβ1-37/Aβ1-42 yielded an AUC of 0.96 and 0.90, respectively. Interestingly, when diminished values of Aβ1-42 in VAD were combined with Aβ1-40 levels (Aβ1-40/Aβ1-42), an AUC of 0.94 could be realized.

### DISCUSSION

### Plasma Aβ peptides in the diagnosis of FTD and VAD

A detailed characterization of Aβ peptide species in plasma of FTD in comparison to VAD, AD and depressive controls (DC) is presented. With regard to Aβ1-40 and Aβ1-42 in AD, the results are in line with previous studies of others (Irizarry 2004, Mehta and Pirttila 2005). In line with Example I, a prominent percentage increase of plasma Aβ1-40 (Aβ1-40%) in VAD as compared to AD and DC was found, which was paralleled by decreases of Aβ1-38%, Aβ1-42% and Aβ2-40%. Most interestingly, FTD revealed alterations similar to VAD with regard to Aβ1-40% and Aβ1-38%. In contrast, FTD displayed lower absolute levels of Aβ1-37 and higher percentage levels of Aβ1-42 than VAD.

Recently, CSF levels of Aβ peptides 1-37 and 1-38 were reported to be decreased in FTD, whereas percentage Aβ1-40 were elevated as compared to non demented controls and AD (Bibl et al., 2007b; Bibl et al., 2007c). It is noteworthy that a very similar pattern of derangement could be shown in the present blood based analysis of Aβ peptides in FTD. However, this is most surprising as the AD specific drop of CSF Aβ1-42 could be retraced in plasma and the relation of CSF to plasma Aβ is not yet clarified.

Anyway, genetic factors are discussed to play an important role for the aetio-pathogenesis of FTD, rather than for the majority of sporadic AD cases. Recently, presenilin1 mutations have been shown to be associated with FTD-like clinical phenotypes, one of which causes a Pick-type tauopathy without the development of amyloid plaques in neuropathological confirmation (Dermaut et al., 2004). Under this aspect, one may speculate that certain genetic alterations of the APP metabolism may predispose an individual to develop FTD. These subsequent changes of the APP metabolism may be reflected by a decrease of carboxyterminally truncated Aβ peptides in CSF as well as in plasma, no matter, whether these alterations represent a causal event in the pathogenesis of neurodegeneration or just an epiphenomenon. For further elucidation of this aspect, especially with regard to CSF and plasma neurochemical phenotypes, we propose to investigate Aβ peptide patterns in affected families and transgenic cell culture models.

In concordance with our results on plasma Aβ peptides in VAD, a recent publication by Gurol and colleagues (2006) reported a correlation between increased Aβ40 and the load of white matter lesions in MCI, AD and CAA. This effect turned out to be independent of potential confounders, such as age, sex, hypertension, diabetes mellitus, APO-E status, homocysteine, folate and B12. Increased Aβ40 was interpreted as a potential cerebrovascular risk factor and taken together with our results, it may also be a potential biomarker, indicating the impact of cerebrovascular injury on a dementia process.

Conversely, the considerably larger Rotterdam study found a comparable risk of developing AD or VAD, respectively, as judged from the preclinical Aβ40 and Aβ42 levels. However, a direct comparison of Aβ40 and Aβ42 among prevalent AD and VAD was not reported in their study and they did not measure Aβ1-38 levels. Moreover, the ELISA they used did not allow separate measurement of N-terminal truncated forms of Aβ (e.g. Aβ2-40). These factors may have contributed to the discrepancies between the two studies.

The disease specific role of Aβ1-40 in VAD is underlined by the fact that this peptide comprises the major component of vascular amyloid plaques, but is less abundant in senile AD plaques (Gravina et al. 1995). Nevertheless, it remains an open question whether the relative reduction of plasma Aβ peptides other than Aβ1-40 (i.e. Aβ1-38, Aβ1-42 and Aβ2-40 in comparison DC) may also be related to cerebrovascular amyloid pathology. However, from the above analysis, the diagnostic power of plasma Aβ1-40 levels will be increased if assessed in relation to Aβ1-38 or Aβ1-42, respectively.

### Remark

To apply the findings to routine diagnostic testing, plasma samples have to be investigated following a standardized pretreatment protocol. The focus should be particularly on the effect of sample storage under distinct conditions (e.g. storage time and temperature) prior to analyses.

### Conclusions

Selected carboxy-terminally truncated amyloid β peptides as parts of the overall plasma Aβ peptide pattern are a biomarker for the improved differential diagnosis of FTD and VAD. Both FTD and VAD may be differentiated from other non-demented patients and other dementias, such as AD, by plasma concentrations of Aβ1-40, Aβ1-38 and Aβ1-37. However discrimination between FTD and VAD should additionally consider plasma Aβ1-42 levels.

### References

Arnold SE, Han LY, Clark CM, Grossman M, Trojanowski JQ. Quantitative neurohistological features of frontotemporal degeneration. Neurobiol Aging 2000; 21: 913-919
Bibl M, Esselmann H, Otto M, Lewczuk P, Cepek L, Rüther E, Komhuber J. Cerebrospinal fluid (CSF) amyloid beta (Aβ) peptide patterns in Alzheimer's disease (AD) patients and non-demented controls depend on sample pre-treatment: Indication of carrier- mediated epitope masking of Aβ peptides. Electrophoresis 2004; 25:2912-2918.
Bibl M, Mollenhauer B, Esselmann H, Lewczuk P, Klafki HW, Sparbier K, Smimov A, Cepek L, Trenkwalder C, Ruther E, Komhuber J, Otto M, Wiltfang J. CSF amyloid-beta-peptides in Alzheimer's disease, dementia with Lewy bodies and Parkinson's disease dementia. Brain 2006; 129: 1177-1187.
Bibl M, Esselmann H, Mollenhauer B, Weniger G, Welge V, Liess M, Lewczuk P, Otto M, Schulz JB, Trenkwalder C, Komhuber J, Wiltfang J. Blood based neurochemical diagnosis of vascular dementia: a pilot study. J Neurochem 2007a; 103:467-74.
Bibl M, Mollenhauer B, Lewczuk P, Esselmann H, Wolf S, Trenkwalder C, Otto M, Stiens G, Rüther E, Kornhuber J, Wiltfang J. Validation of amyloid-β peptides in CSF diagnosis of neurodegenerative dementias. Mol Psy 2007b; 12:671-80.
Blacker D, Albert MS, Bassett SS, Go RC, Harrell LE, Folstein MF. Reliability an validity of NINCDS-ARDA criteria for Alzheimer's disease. The National Institute of Mental Health Genetics Initiative. Arch Neurol 1994; 51: 1198-204.
Blennow K. Cerebrospinal Fluid Protein Biomarkers for Alzheimer's disease. NeuroRx 2004; 1, 213-225.
Citron M, Vigo-Pelfrey C, Teplow DB, Miller C, Schenk D, Johnston J, Winblad B, Venizelos N, Lannfelt L, Selkoe DJ. Excessive production of amyloid beta-protein by peripheral cells of symptomatic and presymptomatic patients carrying the Swedish familial Alzheimer disease mutation. Proc Natl Acad Sci USA JID - 7505876 1994; 91: 11993-11997.
Dermaut B, Kumar-Singh S, Engelborghs S, Theuns J, Rademakers R, Saerens J, Pickut BA, Peeters K, van den Broeck M, Vennekens K, Claes S, Cruts M, Cras P, Martin JJ, Van Broeckhoven C, De Deyn PP. A novel presenilin 1 mutation associated with Pick's disease but not β-amyloid plaques. Ann Neurol. 2004; 55:617-626.
Fischer P, Jellinger K, Gatterer G, Danielczyk W. Prospective neuropathological validation of Hachinski's ischaemic score in dementias. J Neurol Neurosurg Psychiatry 1991; 7, 580-583.
Glenner GG, Wong CW. Alzheimer's disease: initial report of the purification and characterization of a novel cerebrovascular amyloid protein. Biochem Biophys Res Commun 1984; 120: 885-890.
Gravina SA, Ho L, Eckman CB, Long KE, Otvos L, Younkin L, Suzuki N, Younkin SG. Amyloid β protein in Alzheimer's diesease brain. J Biol Chem 1995; 270: 7013-7016.
Gurol ME, Irizarry MC, Smith EE, Raju S, Diaz-Arrastia R, Bottiglieri T, Rosand J, Growdon JH, Greenberg SM. Plasma β-amyloid and white matter lesions in AD, MCI and cerebral amyloid angiopathy. Neurology 2006; 66:23-29.
Haass C, Selkoe DJ. Cellular processing of beta-amyloid precursor protein and the genesis of amyloid beta-peptide. Cell 1993; 75: 1039-1042.
Hulstaert F, Blennow K, Ivanoiu A, Schoonderwald HC, Riemenschneider M, De Deyn PP, Bancher C, Cras P, Wiltfang J, Mehta PD, Iqbal K, Pottel H, Vanmechelen E, Vanderstichele H. Improved discrimination of AD-patients using ß-amyloid (1-42) and tau levels in CSF. Neurology 1999; 52:1555-1562.
Irizarry MC. Biomarkers of Alzheimer disease in plasma. NeuroRX 2004; 1:226-234.
Janek K, Rothemund S, Gast K, Beyermann M, Zipper J, Fabian H, Bienert M, Krause E. Study of the conformational transition of A beta(1-42) using D-amino acid replacement analogues. Biochemistry 2001; 40, 5457-5463.
Kapaki E, Paraskevas GP, Zalonis I, Zournas C. CSF tau protein and beta-amyloid (1-42) in Alzheimers disease diagnosis: discrimination from normal ageing and other dementias in the Greek population. Eur J Neurol 2003; 10(2):119-28.
Lewczuk P, Esselmann H, Bibl M, Paul S, Svitek J, Miertschischk J, Meyrer R, Smimov A, Klein C, Bleich S, Sperling W, Komhuber J, Rüther E, Wiltfang J. Electrophoretic separation of amyloid β (Aβ) peptides in plasma. Electrophoresis 2004(a); 25: 3336-3343.
Lewczuk P, Esselmann H, Groemer TW, Bibl M, Maler JM, Steinacker P, Otto M, Kornhuber J, Wiltfang J. Amyloid beta peptides in cerebrospinal fluid as profiled with surface enhanced laser desorption/ionization time-of-flight mass spectrometry: evidence of novel biomarkers in Alzheimer's disease. Biol Psychiatry 2004b; 55 (5):524 -30 55: 524-530.
McKhann G, Drachman D, Folstein M, Katzmann R, Price D, Stadlan E M. Clinical diagnosis of Alzheimer's disease: report of the NINCDS-ADRDA Work Group under the auspices of Department of Health and Human Services Task Force on Alzheimer's Disease. Neurology 1984; 34, 939-944.
McKhann GM, Albert MS, Grossman M, Miller B, Dickson D, Trojanowski JQ. Clinical and pathological diagnosis of frontotemporal dementia. Report of the work group on frontotemporal dementia and Pick's disease. Arch Neurol 2001; 58:1803-1809.
Mehta P D, Pirttila T. Increased cerebrospinal fluid A beta38/A beta42 in Alzheimer disease. Neurodegener Dis. 2005; 2, 242-245.
Neary D, Snowden JS, Gustafson L, Passant U, Stuss D, Black S, Freedman M, Kertesz A, Robert PH, Albert M, Boone K, Miller BL, Cummings J, Benson DF. Frontotemporal lobar degeneration: a consensus on clinical diagnostic criteria. Neurology 1998; 51: 1546-1554.
Pomara N, Doraiswamy PM, Willoughby LM, Roth AE, Mulsant BH, Sidtis JJ, Mehta PD, Reynolds CF 3rd, Pollock BG. Elevation in plasma Abeta42 in geriatric depression: a pilot study. Neurochem Res 2006; 31:341-9
Roman GC, Tatemichi TK, Erkinjutti T, Cummings JL, Masdeu JC, Garcia JH, Amaducci L, Orgogozo JM, Brun A, Hofmann A. Vascular dementia: diagnostic criteria for research studies: report of the NINDS-AIREN International Workshop. Neurology 1993; 43:243-245.
Riemenschneider M, Wagenpfeil S, Diehl J, Lautenschlager N, Theml T, Heldmann B, Drzezga A, Jahn T, Forstl H, Kurz A. Tau and Abeta42 protein in CSF of patients with frontotemporal degeneration. Neurology 2002; 58: 1622-1628.
Sjogren M, Minthon L, Davidsson P, Granerus A-K, Clarberg A, Vanderstichele H, Vanmechelen E, Wallin A, Blennow K. CSF levels of tau, beta-amyloid(1-42) and GAP-43 in frontotemporal dementia, other types of dementia and normal aging. J Neural Transm 2000; 107: 563-579.
Van Oijen M, Hofmann A, Soares HD, Koudstaal PJ, Breeteler MMB. Plasma Aβ1-40 and Aβ1-42 and the risk of dementia: a prospective case-cohort study. Lancet Neurol 2006; 5:655-660.
Vollmann J, Winau R. Informed consent in human experimentation before the Nuremberg code. BMJ 1996; 313: 1445-1449.
Wiltfang J, Esselmann H, Bibl M, Smimov A, Otto M, Paul S, Schmid B, Klafki H-W, Maler M, Dyrks T, Bienert M, Beyermann M, Ruther E, Kornhuber J. Highly conserved and disease-specific patterns of carboxyterminally truncated Abeta peptides 1-37/38/39 in addition to 1-40/42 in Alzheimer's disease and patients with chronic neuroinflammation. J Neurochem 2002; 81: 481-496.
Wiltfang J, Esselmann H, Cupers P, Neumann M, Kretzschmar H, Beyermann M, Schleuder D, Jahn H, Ruther E, Kornhuber J, Annaert W, De Strooper B, Saftig P. Elevation of Abeta peptide 2-42 in sporadic and familial Alzheimer's disease and its generation in PS1 knockout cells. J Biol Chem 2001.
World Medical Organisation. Declaration of Helsinki. British Medical Journal 1996; 313:1448-1449.

**Table 1**

| **Diagnosis** | **DC (n=13)** | | **AD (n=10)** | | **VAD (n=13)** | |
|---|---|---|---|---|---|---|
| | MW | ±SD | MW | ±SD | MW | ±SD |
| **Age** | 62.62 | 7.68 | 69.60 | 8.86 | 71.00 | 6.73 |
| **MMSE** | 27.83 | 2.86 | 15.90 | 4.93 | 17.91 | 9.20 |
| **CSF Tau ELISA (ng/ml)** | 0.29 | 0.22 | 0.91 | 0.69 | 0.31 | 0.20 |
| **CSF Aβ1-42 ELISA (ng/ml)** | 0.94 | 0.46 | 0.33 | 0.11 | 0.48 | 0.21 |
| **Aβ1-37 (pg/ml)** | 25.30 | 7.33 | 23.60 | 5.40 | 21.10 | 7.07 |
| **Aβ1-38 (pg/ml)** | 33.79 | 13.18 | 32.57 | 12.18 | 30.53 | 11.95 |
| **Aβ1-39 (pg/ml)** | 28.04 | 7.57 | 29.58 | 8.24 | 27.29 | 9.83 |
| **Aβ1-40 (pg/ml)** | 223.6 | 88.3 | 219.4 | 111.3 | 272.2 | 118.1 |
| **Aβ1-42 (pg/ml)** | 26.49 | 7.10 | 24.41 | 4.82 | 18.65 | 8.29 |
| **Aβ2-40 (pg/ml)** | 20.09 | 5.62 | 21.20 | 3.70 | 15.22 | 5.70 |
| **total Aβ (pg/ml) ¹** | 378.0 | 124.2 | 370.0 | 144.5 | 410.6 | 152.9 |
| **Aβ1-37% ²** | 6.89 | 1.07 | 6.76 | 1.28 | 5.46 | 1.49 |
| **Aβ1-38% ²** | 8.84 | 1.12 | 8.82 | 0.38 | 7.43 | 0.85 |
| **Aβ1-39% ²** | 7.63 | 1.12 | 8.35 | 1.21 | 6.73 | 0.69 |
| **Aβ1-40% ²** | 57.64 | 6.54 | 56.89 | 7.36 | 64.74 | 6.12 |
| **Aβ1-42% ²** | 7.24 | 0.99 | 7.17 | 1.89 | 4.65 | 1.69 |
| **Aβ2-40% ²** | 5.52 | 1.15 | 6.32 | 1.92 | 3.82 | 1.02 |
| **Ratio Aβ1-38/1-40** | 0.155 | 0.027 | 0.158 | 0.022 | 0.116 | 0.019 |
| **Ratio Aβ1-42/1-40** | 0.128 | 0.030 | 0.132 | 0.051 | 0.074 | 0.032 |
| **Ratio Aβ2-40/1-40** | 0.099 | 0.031 | 0.117 | 0.052 | 0.060 | 0.020 |

| | | | | | | |
|---|---|---|---|---|---|---|
| ¹total Aβ peptide concentration as measured by the sum of all investigated Aβ peptides, ²percentage abundance of Aβ peptides relative to the sum of all investigated Aβ peptides. | | | | | | |

**Table 2a**

| **cutoff** | **sensitivly** | **specificity** | **youden** |
|---|---|---|---|
| 0.0000 | 0.0000 | 1.0000 | 1.0000 |
| 0.1050 | 0.0330 | 1.0000 | 1.0330 |
| 0.1250 | 0.0670 | 1.0000 | 1.0670 |
| 0.1350 | 0.1000 | 1.0000 | 1.1000 |
| 0.1450 | 0.2000 | 1.0000 | 1.2000 |
| 0.1550 | 0.2670 | 1.0000 | 1.2670 |
| 0.1627 | 0.3330 | 1.0000 | 1.3330 |
| 0.1677 | 0.3330 | 0.9830 | 1.3160 |
| 0.1750 | 0.4330 | 0.9670 | 1.4000 |
| 0.1850 | 0.5670 | 0.9670 | 1.5340 |
| 0.1950 | 0.6670 | 0.9670 | 1.6340 |
| 0.2021 | 0.7670 | 0.9330 | 1.7000 |
| 0.2071 | 0.7670 | 0.9170 | 1.6840 |
| 0.2150 | 0.8670 | 0.8830 | 1.7500 |
| 0.2250 | 0.8670 | 0.8170 | 1.6840 |
| 0.2307 | 0.9000 | 0.6830 | 1.5830 |
| 0.2357 | 0.9000 | 0.6670 | 1.5670 |
| 0.2402 | 0.9000 | 0.5830 | 1.4830 |
| 0.2452 | 0.9000 | 0.5670 | 1.4670 |
| 0.2550 | 0.9330 | 0.5000 | 1.4330 |
| 0.2650 | 0.9670 | 0.4170 | 1.3840 |
| 0.2750 | 0.9670 | 0.4000 | 1.3670 |
| 0.2819 | 0.9670 | 0.3000 | 1.2670 |
| 0.2869 | 0.9670 | 0.2830 | 1.2500 |
| 0.3000 | 0.9670 | 0.2500 | 1.2170 |
| 0.3150 | 1.0000 | 0.2000 | 1.2000 |
| 0.3250 | 1.0000 | 0.1170 | 1.1170 |
| 0.3350 | 1.0000 | 0.0830 | 1.0830 |
| 0.3450 | 1.0000 | 0.0670 | 1.0670 |
| 0.3600 | 1.0000 | 0.0500 | 1.0500 |
| 0.4050 | 1.0000 | 0.0330 | 1.0330 |
| 0.5050 | 1.0000 | 0.0170 | 1.0170 |
| 1.0000 | 1.0000 | 0.0000 | 1.0000 |

**Table 2b**

| **cutoff** | **sensitivly** | **specificity** | **youden** |
|---|---|---|---|
| 0.0000 | 0.0000 | 1.0000 | 1.0000 |
| 0.1050 | 0.0330 | 1.0000 | 1.0330 |
| 0.1250 | 0.0670 | 1.0000 | 1.0670 |
| 0.1350 | 0.1000 | 1.0000 | 1.1000 |
| 0.1450 | 0.2000 | 1.0000 | 1.2000 |
| 0.1550 | 0.2670 | 1.0000 | 1.2670 |
| 0.1627 | 0.3330 | 1.0000 | 1.3330 |
| 0.1677 | 0.3330 | 0.9670 | 1.3000 |
| 0.1750 | 0.4330 | 0.9670 | 1.4000 |
| 0.1850 | 0.5670 | 0.9670 | 1.5340 |
| 0.1950 | 0.6670 | 0.9670 | 1.6340 |
| 0.2021 | 0.7670 | 0.9000 | 1.6670 |
| 0.2071 | 0.7670 | 0.8670 | 1.6340 |
| 0.2150 | 0.8670 | 0.8670 | 1.7340 |
| 0.2250 | 0.8670 | 0.8330 | 1.7000 |
| 0.2307 | 0.9000 | 0.6330 | 1.5330 |
| 0.2357 | 0.9000 | 0.6000 | 1.5000 |
| 0.2402 | 0.9000 | 0.5330 | 1.4330 |
| 0.2452 | 0.9000 | 0.5000 | 1.4000 |
| 0.2550 | 0.9330 | 0.4670 | 1.4000 |
| 0.2650 | 0.9670 | 0.4000 | 1.3670 |
| 0.2750 | 0.9670 | 0.3670 | 1.3340 |
| 0.2819 | 0.9670 | 0.3000 | 1.2670 |
| 0.2869 | 0.9670 | 0.2670 | 1.2340 |
| 0.3000 | 0.9670 | 0.2330 | 1.2000 |
| 0.3150 | 1.0000 | 0.2330 | 1.2330 |
| 0.3250 | 1.0000 | 0.1330 | 1.1330 |
| 0.3500 | 1.0000 | 0.0670 | 1.0670 |
| 0.4050 | 1.0000 | 0.0330 | 1.0330 |
| 1.0000 | 1.0000 | 0.0000 | 1.0000 |

**Table 2c**

| **cutoff** | **sensitiviy** | **specificity** | **youden** |
|---|---|---|---|
| 0.0000 | 0.0000 | 1.0000 | 1.0000 |
| 0.1050 | 0.0330 | 1.0000 | 1.0330 |
| 0.1250 | 0.0670 | 1.0000 | 1.0670 |
| 0.1350 | 0.1000 | 1.0000 | 1.1000 |
| 0.1450 | 0.2000 | 1.0000 | 1.2000 |
| 0.1550 | 0.2670 | 1.0000 | 1.2670 |
| 0.1650 | 0.3330 | 1.0000 | 1.3330 |
| 0.1750 | 0.4330 | 0.9670 | 1.4000 |
| 0.1850 | 0.5670 | 0.9670 | 1.5340 |
| 0.1950 | 0.6670 | 0.9670 | 1.6340 |
| 0.2050 | 0.7670 | 0.9670 | 1.7340 |
| 0.2150 | 0.8670 | 0.9000 | 1.7670 |
| 0.2250 | 0.8670 | 0.8000 | 1.6670 |
| 0.2350 | 0.9000 | 0.7330 | 1.6330 |
| 0.2450 | 0.9000 | 0.6330 | 1.5330 |
| 0.2550 | 0.9330 | 0.5330 | 1.4660 |
| 0.2700 | 0.9670 | 0.4330 | 1.4000 |
| 0.2850 | 0.9670 | 0.3000 | 1.2670 |
| 0.3000 | 0.9670 | 0.2670 | 1.2340 |
| 0.3150 | 1.0000 | 0.1670 | 1.1670 |
| 0.3300 | 1.0000 | 0.1000 | 1.1000 |
| 0.3450 | 1.0000 | 0.0670 | 1.0670 |
| 0.4600 | 1.0000 | 0.0330 | 1.0330 |
| 1.0000 | 1.0000 | 0.0000 | 1.0000 |

**Table 3a**

| **cutoff** | **sensitivly** | **specificity** | **youden** |
|---|---|---|---|
| 0.0000 | 0.0000 | 1.0000 | 1.0000 |
| 0.0650 | 0.0330 | 1.0000 | 1.0330 |
| 0.0750 | 0.1670 | 1.0000 | 1.1670 |
| 0.0850 | 0.3000 | 0.9830 | 1.2830 |
| 0.0933 | 0.4330 | 0.9500 | 1.3830 |
| 0.0983 | 0.4330 | 0.9330 | 1.3660 |
| 0.1036 | 0.6000 | 0.8670 | 1.4670 |
| 0.1086 | 0.6000 | 0.8500 | 1.4500 |
| 0.1107 | 0.7000 | 0.7670 | 1.4670 |
| 0.1148 | 0.7000 | 0.7500 | 1.4500 |
| 0.1191 | 0.7000 | 0.7330 | 1.4330 |
| 0.1250 | 0.8330 | 0.6670 | 1.5000 |
| 0.1338 | 0.8670 | 0.6170 | 1.4840 |
| 0.1388 | 0.8670 | 0.6000 | 1.4670 |
| 0.1450 | 0.9000 | 0.5670 | 1.4670 |
| 0.1550 | 0.9000 | 0.4670 | 1.3670 |
| 0.1650 | 0.9000 | 0.3500 | 1.2500 |
| 0.1750 | 0.9670 | 0.3000 | 1.2670 |
| 0.1850 | 1.0000 | 0.2330 | 1.2330 |
| 0.1950 | 1.0000 | 0.1670 | 1.1670 |
| 0.2050 | 1.0000 | 0.1000 | 1.1000 |
| 0.2150 | 1.0000 | 0.0830 | 1.0830 |
| 0.2400 | 1.0000 | 0.0670 | 1.0670 |
| 0.2700 | 1.0000 | 0.0500 | 1.0500 |
| 0.2850 | 1.0000 | 0.0330 | 1.0330 |
| 0.3100 | 1.0000 | 0.0170 | 1.0170 |
| 1.0000 | 1.0000 | 0.0000 | 1.0000 |

**Table 3b**

| **cutoff** | **sensitiviy** | **specificity** | **youden** |
|---|---|---|---|
| 0.0000 | 0.0000 | 1.0000 | 1.0000 |
| 0.0650 | 0.0330 | 1.0000 | 1.0330 |
| 0.0750 | 0.1670 | 1.0000 | 1.1670 |
| 0.0850 | 0.3000 | 1.0000 | 1.3000 |
| 0.0933 | 0.4330 | 0.9670 | 1.4000 |
| 0.0983 | 0.4330 | 0.9330 | 1.3660 |
| 0.1036 | 0.6000 | 0.8670 | 1.4670 |
| 0.1086 | 0.6000 | 0.8330 | 1.4330 |
| 0.1107 | 0.7000 | 0.7000 | 1.4000 |
| 0.1148 | 0.7000 | 0.6670 | 1.3670 |
| 0.1191 | 0.7000 | 0.6330 | 1.3330 |
| 0.1250 | 0.8330 | 0.6000 | 1.4330 |
| 0.1338 | 0.8670 | 0.5670 | 1.4340 |
| 0.1388 | 0.8670 | 0.5330 | 1.4000 |
| 0.1450 | 0.9000 | 0.5330 | 1.4330 |
| 0.1550 | 0.9000 | 0.4330 | 1.3330 |
| 0.1650 | 0.9000 | 0.3000 | 1.2000 |
| 0.1750 | 0.9670 | 0.2330 | 1.2000 |
| 0.1850 | 1.0000 | 0.1670 | 1.1670 |
| 0.1950 | 1.0000 | 0.1330 | 1.1330 |
| 0.2650 | 1.0000 | 0.0330 | 1.0330 |
| 1.0000 | 1.0000 | 0.0000 | 1.0000 |

**Table 3c**

| **cutoff** | **sensitiviy** | **specificity** | **youden** |
|---|---|---|---|
| 0.0000 | 0.0000 | 1.0000 | 1.0000 |
| 0.0650 | 0.0330 | 1.0000 | 1.0330 |
| 0.0750 | 0.1670 | 1.0000 | 1.1670 |
| 0.0850 | 0.3000 | 0.9670 | 1.2670 |
| 0.0950 | 0.4330 | 0.9330 | 1.3660 |
| 0.1050 | 0.6000 | 0.8670 | 1.4670 |
| 0.1150 | 0.7000 | 0.8330 | 1.5330 |
| 0.1250 | 0.8330 | 0.7330 | 1.5660 |
| 0.1350 | 0.8670 | 0.6670 | 1.5340 |
| 0.1450 | 0.9000 | 0.6000 | 1.5000 |
| 0.1550 | 0.9000 | 0.5000 | 1.4000 |
| 0.1650 | 0.9000 | 0.4000 | 1.3000 |
| 0.1750 | 0.9670 | 0.3670 | 1.3340 |
| 0.1850 | 1.0000 | 0.3000 | 1.3000 |
| 0.1950 | 1.0000 | 0.2000 | 1.2000 |
| 0.2050 | 1.0000 | 0.1670 | 1.1670 |
| 0.2150 | 1.0000 | 0.1330 | 1.1330 |
| 0.2400 | 1.0000 | 0.1000 | 1.1000 |
| 0.2700 | 1.0000 | 0.0670 | 1.0670 |
| 0.2850 | 1.0000 | 0.0330 | 1.0330 |
| 1.0000 | 1.0000 | 0.0000 | 1.0000 |

**Table 4b**

| **Positive if Greater Than or Equal To** | **Sensitivity** | **1** - **Specificity** |
|---|---|---|
| -.8500 | 1.000 | 1.000 |
| .1600 | 1.000 | .967 |
| .1750 | 1.000 | .933 |
| .1880 | 1.000 | .867 |
| .2030 | 1.000 | .833 |
| .2150 | 1.000 | .800 |
| .2250 | 1.000 | .767 |
| .2350 | 1.000 | .733 |
| .2450 | 1.000 | .700 |
| .2550 | 1.000 | .667 |
| .2750 | 1.000 | .567 |
| .2910 | 1.000 | .533 |
| .2960 | 1.000 | .500 |
| .3050 | 1.000 | .467 |
| .3150 | 1.000 | .433 |
| .3250 | 1.000 | .300 |
| .3315 | 1.000 | .200 |
| .3365 | 1.000 | .167 |
| .3450 | .967 | .167 |
| .3650 | .967 | .133 |
| .3850 | .967 | .100 |
| .4000 | .933 | .100 |
| .4150 | .933 | .067 |
| .4450 | .933 | .033 |
| .5100 | .900 | .000 |
| .5600 | .867 | .000 |
| .5900 | .833 | .000 |
| .6250 | .800 | .000 |
| .6500 | .767 | .000 |
| .6700 | .733 | .000 |
| .6850 | .700 | .000 |
| .6950 | .667 | .000 |
| .7100 | .600 | .000 |
| .7250 | .567 | .000 |
| .7350 | .533 | .000 |
| .7450 | .500 | .000 |
| .7650 | .433 | .000 |
| .8100 | .400 | .000 |
| .8450 | .367 | .000 |
| .8600 | .300 | .000 |
| .8900 | .233 | .000 |
| .9450 | .200 | .000 |
| .9950 | .167 | .000 |
| 1.0450 | .133 | .000 |
| 1.1100 | .100 | .000 |
| 1.2650 | .067 | .000 |
| 1.4000 | .033 | .000 |
| 2.4100 | .000 | .000 |

**Table 4a**

| **Positive if Greater Than or Equal To** | **Sensitivity** | **1 - Specificity** |
|---|---|---|
| -.8500 | 1.000 | 1.000 |
| .1600 | 1.000 | .983 |
| .1750 | 1.000 | .967 |
| .1880 | 1.000 | .933 |
| .2030 | 1.000 | .917 |
| .2150 | 1.000 | .900 |
| .2250 | 1.000 | .883 |
| .2350 | 1.000 | .867 |
| .2450 | 1.000 | .850 |
| .2550 | 1.000 | .833 |
| .2750 | 1.000 | .783 |
| .2910 | 1.000 | .767 |
| .2960 | 1.000 | .750 |
| .3050 | 1.000 | .733 |
| .3150 | 1.000 | .717 |
| .3250 | 1.000 | .650 |
| .3315 | 1.000 | .600 |
| .3365 | 1.000 | .583 |
| .3450 | .967 | .583 |
| .3650 | .967 | .567 |
| .3850 | .967 | .550 |
| .4000 | .933 | .550 |
| .4150 | .933 | .533 |
| .4300 | .933 | .517 |
| .4550 | .933 | .500 |
| .4750 | .900 | .483 |
| .5050 | .900 | .467 |
| .5400 | .900 | .450 |
| .5550 | .867 | .433 |
| .5650 | .867 | .417 |
| .5750 | .833 | .417 |
| .5850 | .833 | .400 |
| .6000 | .833 | .367 |
| .6250 | .800 | .367 |
| .6500 | .767 | .367 |
| .6650 | .733 | .367 |
| .6750 | .733 | .350 |
| .6850 | .700 | .350 |
| .6950 | .667 | .350 |
| .7100 | .600 | .333 |
| .7250 | .567 | .333 |
| .7350 | .533 | .317 |
| .7450 | .500 | .300 |
| .7550 | .433 | .300 |
| .7700 | .433 | .267 |
| .7850 | .400 | .267 |
| .8000 | .400 | .250 |
| .8150 | .400 | .233 |
| .8300 | .400 | .217 |
| .8450 | .367 | .217 |
| .8600 | .300 | .217 |
| .8900 | .233 | .200 |
| .9250 | .200 | .183 |
| .9500 | .200 | .150 |
| .9700 | .200 | .133 |
| .9950 | .167 | .117 |
| 1.0350 | .133 | .117 |
| 1.0650 | .133 | .100 |
| 1.0750 | .133 | .083 |
| 1.1000 | .100 | .067 |
| 1.1300 | .100 | .050 |
| 1.1500 | .067 | .050 |
| 1.2000 | .067 | .033 |
| 1.3150 | .067 | .017 |
| 1.4000 | .033 | .017 |
| 1.4500 | .000 | .017 |
| 2.4900 | .000 | .000 |

**Table 4c**

| **Positive if Greater Than or Equal To** | **Sensitivity** | **1** - **Specificity** |
|---|---|---|
| -.6600 | .000 | .000 |
| .3650 | .033 | .000 |
| .4150 | .067 | .000 |
| .4550 | .067 | .033 |
| .4750 | .100 | .033 |
| .5050 | .100 | .067 |
| .5400 | .100 | .100 |
| .5550 | .133 | .133 |
| .5650 | .133 | .167 |
| .5750 | .167 | .167 |
| .5850 | .167 | .200 |
| .6000 | .167 | .267 |
| .6250 | .200 | .267 |
| .6500 | .233 | .267 |
| .6650 | .267 | .267 |
| .6750 | .267 | .300 |
| .6850 | .300 | .300 |
| .6950 | .333 | .300 |
| .7100 | .400 | .333 |
| .7250 | .433 | .333 |
| .7350 | .467 | .367 |
| .7450 | .500 | .400 |
| .7550 | .567 | .400 |
| .7700 | .567 | .467 |
| .7850 | .600 | .467 |
| .8000 | .600 | .500 |
| .8150 | .600 | .533 |
| .8300 | .600 | .567 |
| .8450 | .633 | .567 |
| .8600 | .700 | .567 |
| .8900 | .767 | .600 |
| .9250 | .800 | .633 |
| .9500 | .800 | .700 |
| .9700 | .800 | .733 |
| .9950 | .833 | .767 |
| 1.0350 | .867 | .767 |
| 1.0650 | .867 | .800 |
| 1.0750 | .867 | .833 |
| 1.1000 | .900 | .867 |
| 1.1300 | .900 | .900 |
| 1.1500 | .933 | .900 |
| 1.2000 | .933 | .933 |
| 1.3150 | .933 | .967 |
| 1.4000 | .967 | .967 |
| 1.4500 | 1.000 | .967 |
| 2.4900 | 1.000 | 1.000 |
| | | |
| | | |

**Table 4d**

| **Positive if Less Than or Equal To** | **Sensitivity** | **1 - Specificity** |
|---|---|---|
| -.8500 | .000 | .000 |
| .1600 | .033 | .000 |
| .1750 | .067 | .000 |
| .1880 | .133 | .000 |
| .2030 | .167 | .000 |
| .2150 | .200 | .000 |
| .2250 | .233 | .000 |
| .2350 | .267 | .000 |
| .2450 | .300 | .000 |
| .2550 | .333 | .000 |
| .2750 | .433 | .000 |
| .2910 | .467 | .000 |
| .2960 | .500 | .000 |
| .3050 | .533 | .000 |
| .3150 | .567 | .000 |
| .3250 | .700 | .000 |
| .3315 | .800 | .000 |
| .3415 | .833 | .000 |
| .3650 | .867 | .000 |
| .3950 | .900 | .000 |
| .4150 | .933 | .000 |
| .4300 | .967 | .000 |
| .4550 | .967 | .033 |
| .4750 | 1.000 | .033 |
| .5050 | 1.000 | .067 |
| .5400 | 1.000 | .100 |
| .5550 | 1.000 | .133 |
| .5700 | 1.000 | .167 |
| .5850 | 1.000 | .200 |
| .6300 | 1.000 | .267 |
| .6850 | 1.000 | .300 |
| .7150 | 1.000 | .333 |
| .7350 | 1.000 | .367 |
| .7500 | 1.000 | .400 |
| .7750 | 1.000 | .467 |
| .8000 | 1.000 | .500 |
| .8150 | 1.000 | .533 |
| .8450 | 1.000 | .567 |
| .8900 | 1.000 | .600 |
| .9250 | 1.000 | .633 |
| .9500 | 1.000 | .700 |
| .9700 | 1.000 | .733 |
| 1.0200 | 1.000 | .767 |
| 1.0650 | 1.000 | .800 |
| 1.0750 | 1.000 | .833 |
| 1.1000 | 1.000 | .867 |
| 1.1400 | 1.000 | .900 |
| 1.2000 | 1.000 | .933 |
| 1.3650 | 1.000 | .967 |
| 2.4900 | 1.000 | 1.000 |

**Table 5b**

| **Positive if Greater Than or Equal To** | **Sensitivity** | **1** - **Specificity** |
|---|---|---|
| -.7300 | 1.000 | 1.000 |
| .2900 | 1.000 | .967 |
| .3350 | 1.000 | .933 |
| .3625 | 1.000 | .900 |
| .3725 | 1.000 | .867 |
| .3850 | 1.000 | .833 |
| .4060 | 1.000 | .800 |
| .4260 | 1.000 | .767 |
| .4350 | 1.000 | .733 |
| .4550 | 1.000 | .700 |
| .4750 | 1.000 | .633 |
| .4850 | 1.000 | .600 |
| .5000 | 1.000 | .533 |
| .5150 | 1.000 | .500 |
| .5250 | 1.000 | .467 |
| .5350 | 1.000 | .433 |
| .5450 | 1.000 | .400 |
| .5550 | 1.000 | .367 |
| .5655 | 1.000 | .267 |
| .5865 | 1.000 | .233 |
| .6110 | 1.000 | .200 |
| .6310 | 1.000 | .167 |
| .6460 | 1.000 | .133 |
| .6650 | 1.000 | .100 |
| .7050 | 1.000 | .067 |
| .7550 | 1.000 | .033 |
| .7850 | .967 | .033 |
| .8200 | .933 | .033 |
| .8800 | .933 | .000 |
| .9200 | .900 | .000 |
| .9350 | .867 | .000 |
| .9500 | .833 | .000 |
| .9950 | .800 | .000 |
| 1.0600 | .767 | .000 |
| 1.1050 | .733 | .000 |
| 1.1550 | .667 | .000 |
| 1.1950 | .633 | .000 |
| 1.2200 | .567 | .000 |
| 1.2850 | .533 | .000 |
| 1.3700 | .500 | .000 |
| 1.4150 | .467 | .000 |
| 1.4350 | .433 | .000 |
| 1.5050 | .400 | .000 |
| 1.5750 | .367 | .000 |
| 1.6000 | .333 | .000 |
| 1.6250 | .300 | .000 |
| 1.6550 | .267 | .000 |
| 1.6750 | .233 | .000 |
| 1.8250 | .167 | .000 |
| 2.0450 | .133 | .000 |
| 2.1300 | .100 | .000 |
| 2.1650 | .067 | .000 |
| 2.2150 | .033 | .000 |
| 3.2400 | .000 | .000 |

**Table 6a**

| **Positive if Greater Than or Equal To** | **Sensitivity** | **1 - Specificity** |
|---|---|---|
| -.7300 | 1.000 | 1.000 |
| .2900 | 1.000 | .983 |
| .3350 | 1.000 | .967 |
| .3625 | 1.000 | .950 |
| .3725 | 1.000 | .933 |
| .3850 | 1.000 | .917 |
| .4060 | 1.000 | .900 |
| .4260 | 1.000 | .883 |
| .4350 | 1.000 | .867 |
| .4550 | 1.000 | .850 |
| .4750 | 1.000 | .817 |
| .4850 | 1.000 | .800 |
| .5000 | 1.000 | .767 |
| .5150 | 1.000 | .750 |
| .5250 | 1.000 | .733 |
| .5350 | 1.000 | .717 |
| .5450 | 1.000 | .700 |
| .5550 | 1.000 | .683 |
| .5655 | 1.000 | .633 |
| .5865 | 1.000 | .617 |
| .6110 | 1.000 | .600 |
| .6310 | 1.000 | .583 |
| .6460 | 1.000 | .567 |
| .6650 | 1.000 | .550 |
| .7050 | 1.000 | .533 |
| .7550 | 1.000 | .517 |
| .7850 | .967 | .517 |
| .8200 | .933 | .517 |
| .8550 | .933 | .467 |
| .8650 | .933 | .450 |
| .8900 | .933 | .433 |
| .9150 | .900 | .417 |
| .9250 | .900 | .400 |
| .9350 | .867 | .400 |
| .9500 | .833 | .400 |
| .9650 | .800 | .400 |
| .9850 | .800 | .383 |
| 1.0150 | .800 | .367 |
| 1.0600 | .767 | .367 |
| 1.1050 | .733 | .367 |
| 1.1500 | .667 | .367 |
| 1.1850 | .667 | .350 |
| 1.1950 | .633 | .350 |
| 1.2200 | .567 | .350 |
| 1.2450 | .533 | .350 |
| 1.2600 | .533 | .317 |
| 1.2850 | .533 | .300 |
| 1.3150 | .533 | .283 |
| 1.3400 | .500 | .283 |
| 1.3600 | .500 | .267 |
| 1.3750 | .500 | .250 |
| 1.3950 | .500 | .233 |
| 1.4150 | .467 | .217 |
| 1.4350 | .433 | .217 |
| 1.4700 | .400 | .217 |
| 1.5250 | .400 | .200 |
| 1.5750 | .367 | .167 |
| 1.6000 | .333 | .167 |
| 1.6250 | .300 | .167 |
| 1.6550 | .267 | .167 |
| 1.6750 | .233 | .150 |
| 1.7200 | .167 | .150 |
| 1.7650 | .167 | .133 |
| 1.7850 | .167 | .117 |
| 1.8350 | .167 | .100 |
| 1.8850 | .167 | .083 |
| 1.9350 | .167 | .067 |
| 2.0300 | .133 | .067 |
| 2.1050 | .133 | .050 |
| 2.1250 | .100 | .033 |
| 2.1350 | .100 | .017 |
| 2.1650 | .067 | .017 |
| 2.2150 | .033 | .017 |
| 2.2600 | .000 | .017 |
| 3.2800 | .000 | .000 |

**Table 5c**

| **Positive if Greater Than or Equal To** | **Sensitivity** | **1** - **Specificity** |
|---|---|---|
| -.2200 | .000 | .000 |
| .7850 | .033 | .000 |
| .8200 | .067 | .000 |
| .8550 | .067 | .067 |
| .8650 | .067 | .100 |
| .8900 | .067 | .133 |
| .9150 | .100 | .167 |
| .9250 | .100 | .200 |
| .9350 | .133 | .200 |
| .9500 | .167 | .200 |
| .9650 | .200 | .200 |
| .9850 | .200 | .233 |
| 1.0150 | .200 | .267 |
| 1.0600 | .233 | .267 |
| 1.1050 | .267 | .267 |
| 1.1500 | .333 | .267 |
| 1.1850 | .333 | .300 |
| 1.1950 | .367 | .300 |
| 1.2200 | .433 | .300 |
| 1.2450 | .467 | .300 |
| 1.2600 | .467 | .367 |
| 1.2850 | .467 | .400 |
| 1.3150 | .467 | .433 |
| 1.3400 | .500 | .433 |
| 1.3600 | .500 | .467 |
| 1.3750 | .500 | .500 |
| 1.3950 | .500 | .533 |
| 1.4150 | .533 | .567 |
| 1.4350 | .567 | .567 |
| 1.4700 | .600 | .567 |
| 1.5250 | .600 | .600 |
| 1.5750 | .633 | .667 |
| 1.6000 | .667 | .667 |
| 1.6250 | .700 | .667 |
| 1.6550 | .733 | .667 |
| 1.6750 | .767 | .700 |
| 1.7200 | .833 | .700 |
| 1.7650 | .833 | .733 |
| 1.7850 | .833 | .767 |
| 1.8350 | .833 | .800 |
| 1.8850 | .833 | .833 |
| 1.9350 | .833 | .867 |
| 2.0300 | .867 | .867 |
| 2.1050 | .867 | .900 |
| 2.1250 | .900 | .933 |
| 2.1350 | .900 | .967 |
| 2.1650 | .933 | .967 |
| 2.2150 | .967 | .967 |

**Table 5d**

| **Positive if Less Than or Equal To** | **Sensitivity** | **1 - Specificity** |
|---|---|---|
| -.7300 | .000 | .000 |
| .2900 | .033 | .000 |
| .3350 | .067 | .000 |
| .3625 | .100 | .000 |
| .3725 | .133 | .000 |
| .3850 | .167 | .000 |
| .4060 | .200 | .000 |
| .4260 | .233 | .000 |
| .4350 | .267 | .000 |
| .4550 | .300 | .000 |
| .4750 | .367 | .000 |
| .4850 | .400 | .000 |
| .5000 | .467 | .000 |
| .5150 | .500 | .000 |
| .5250 | .533 | .000 |
| .5350 | .567 | .000 |
| .5450 | .600 | .000 |
| .5550 | .633 | .000 |
| .5655 | .733 | .000 |
| .5865 | .767 | .000 |
| .6110 | .800 | .000 |
| .6310 | .833 | .000 |
| .6460 | .867 | .000 |
| .6650 | .900 | .000 |
| .7050 | .933 | .000 |
| .7900 | .967 | .000 |
| .8550 | 1.000 | .067 |
| .8650 | 1.000 | .100 |
| .8900 | 1.000 | .133 |
| .9150 | 1.000 | .167 |
| .9450 | 1.000 | .200 |
| .9850 | 1.000 | .233 |
| 1.0900 | 1.000 | .267 |
| 1.2150 | 1.000 | .300 |
| 1.2600 | 1.000 | .367 |
| 1.2850 | 1.000 | .400 |
| 1.3250 | 1.000 | .433 |
| 1.3600 | 1.000 | .467 |
| 1.3750 | 1.000 | .500 |
| 1.3950 | 1.000 | .533 |
| 1.4500 | 1.000 | .567 |
| 1.5250 | 1.000 | .600 |
| 1.6150 | 1.000 | .667 |
| 1.7150 | 1.000 | .700 |
| 1.7650 | 1.000 | .733 |
| 1.7850 | 1.000 | .767 |
| 1.8350 | 1.000 | .800 |
| 1.8850 | 1.000 | .833 |
| 2.2600 | 1.000 | .967 |
| 3.2800 | 1.000 | 1.000 |
| 1.9950 | 1.000 | .867 |
| 2.1050 | 1.000 | .900 |
| 2.1250 | 1.000 | .933 |
| 2.2050 | 1.000 | .967 |
| 3.2800 | 1.000 | 1.000 |

**Table 6**

| **Diagnosis** | **NDC (n=30)** | | **AD (n=30)** | | **FTLD (n=30)** | |
|---|---|---|---|---|---|---|
| | MW | ±SD | MW | ±SD | MW | ±SD |
| **age** | 61.5 | 11.09 | 65.43 | 7.29 | 61.6 | 11.48 |
| **MMSE** | 28.5 | 1.47 | 19.31 | 5.44 | 20.37 | 8.96 |
| **Tau ELISA** | 0.23 | 0.13 | 0.73 | 0.41 | 0.32 | 0.26 |
| **Aβ₁₋₄₂ ELISA** | 0.86 | 0.28 | 0.37 | 0.13 | 0.70 | 0.20 |
| **Aβ₁₋₄₂/ tau¹** | 4.82 | 2.81 | 0.65 | 0.34 | 3.49 | 1.81 |
| **Aβ₁₋₃₇ (ng/mL)** | 1.36 | 0.49 | 1.21 | 0.47 | 0.90 | 0.42 |
| **Aβ₁₋₃₈ (ng/mL)** | 2.26 | 0.60 | 2.17 | 0.81 | 1.60 | 0.72 |
| **Aβ₁₋₃₉ (ng/mL)** | 1.27 | 0.38 | 1.28 | 0.44 | 1.17 | 0.46 |
| **Aβ₁₋₄₀ (ng/mL)** | 8.42 | 1.98 | 8.27 | 2.81 | 8.67 | 2.43 |
| **Aβ₁₋₄₂ (ng/mL)** | 1.89 | 0.72 | 0.60 | 0.23 | 1.23 | 0.58 |
| **total Aβ (ng/mL) ²** | 15.32 | 3.55 | 13.68 | 4.45 | 13.67 | 4.25 |
| **Aβ₁₋₃₇% ³** | 8.71 | 1.88 | 8.80 | 1.88 | 6.47 | 1.49 |
| **Aβ₁₋₃₈% ³** | 14.79 | 2.09 | 15.74 | 2.09 | 11.49 | 2.09 |
| **Aβ₁₋₃₉% ³** | 8.23 | 1.39 | 9.35 | 1.63 | 8.41 | 1.53 |
| **Aβ₁₋₄₀% ³** | 55.36 | 6.28 | 60.58 | 5.18 | 64.04 | 4.45 |

| | | | | | | |
|---|---|---|---|---|---|---|
| ¹ratio of absolute Aβ 1-42 and tau levels as measured by ELISA; ²total Aβ peptide concentration as measured by the sum of all investigated Aβ peptides; ³percentage abundance of Aβ peptides relative to the sum of all investigated Aβ peptides. | | | | | | |

**Table 7**

| **Diagnosis** | **NDC (n=71)** | | **AD (n=71)** | | **DLB (n=32)** | | **FTD (n=36)** | | **OD (n=93)** | |
|---|---|---|---|---|---|---|---|---|---|---|
| | MW | ±SD | MW | ±SD | MW | ±SD | MW | ±SD | MW | ±SD |
| **age** | 62.70 | 9.89 | 70.14 | 8.91 | 69.94 | 7.43 | 61.58 | 10.33 | 68.86 | 8.96 |
| **MMSE** | 28.59 | 1.42 | 18.19 | 6.42 | 18.15 | 5.42 | 19.10 | 10.29 | 17.05 | 8.85 |
| **Aβ1-37** | 1.15 | 0.51 | 1.01 | 0.49 | 0.75 | 0.39 | 0.78 | 0.37 | 0.90 | 0.45 |
| **Aβ1-38** | 2.12 | 0.72 | 1.93 | 0.71 | 1.34 | 0.69 | 1.36 | 0.62 | 1.62 | 0.72 |
| **Aβ1-38 (MSD)¹** | 0.81 | 0.27 | 0.90 | 0.29 | 0.71 | 0.02 | 0.67 | 0.21 | 0.69 | 0.24 |
| **Aβ1-39** | 1.22 | 0.48 | 1.20 | 0.47 | 0.87 | 0.48 | 1.03 | 0.54 | 1.05 | 0.50 |
| **Aβ1-40** | 8.15 | 2.58 | 7.80 | 2.55 | 5.83 | 2.54 | 7.55 | 2.57 | 6.81 | 2.62 |
| **Aβ1-40^{ox}** | 0.11 | 0.09 | 0.13 | 0.07 | 0.20 | 0.19 | 0.10 | 0.06 | 0.13 | 0.11 |
| **Aβ1-42** | 1.59 | 0.74 | 0.56 | 0.22 | 0.69 | 0.48 | 1.02 | 0.54 | 1.01 | 0.61 |
| **total Aβ²** | 14.34 | 4.66 | 12.63 | 4.19 | 9.67 | 4.58 | 11.83 | 4.40 | 11.52 | 4.74 |
| **Aβ1-37³** | 7.84 | 1.76 | 7.70 | 2.12 | 7.51 | 1.32 | 6.54 | 1.48 | 7.64 | 1.34 |
| **Aβ1-38³** | 14.74 | 1.72 | 15.20 | 1.71 | 13.66 | 1.64 | 11.24 | 1.86 | 13.91 | 1.52 |
| **Aβ1-39³** | 8.44 | 1.56 | 9.43 | 1.66 | 8.80 | 1.16 | 8.36 | 1.79 | 9.00 | 1.45 |
| **Aβ1-40³** | 57.39 | 5.33 | 62.12 | 3.96 | 61.43 | 4.13 | 64.63 | 4.79 | 59.81 | 3.81 |
| **Aβ1-40^{ox 3}** | 0.75 | 0.46 | 1.10 | 0.64 | 1.88 | 0.87 | 0.82 | 0.44 | 1.17 | 0.72 |
| **Aβ1-42³** | 10.84 | 3.14 | 4.45 | 0.96 | 6.72 | 2.26 | 8.43 | 2.40 | 8.49 | 2.67 |

| | | | | | | | | | | |
|---|---|---|---|---|---|---|---|---|---|---|
| ¹ Electrochemiluminescence detection (MSD), subgroup of patients: NDC (n=37), AD (n=31), DLB (n=2) OD (n=47) and FTD (n=33). ² total Aβ peptide concentration; ³percentage abundance of Aβ peptides relative to the total Aβ peptide concentration. | | | | | | | | | | |

**Table 8**

| **Differential diagnosis** | **Parameter** | **Cut off** | **Sensitivity** | **Specificity** | **Youden Index** |
|---|---|---|---|---|---|
| AD versus NDC | ELISA Aβ₁₋₄₂/ tau | 1.383 | 100% | 96% | 1.96 |
| | Aβ ₁₋₄₂/ Aβ ₁₋₃₈ | 0.475 | 100% | 97% | 1.97 |
| FTLD versus NDC | ELISA Aβ₁₋₄₂/ tau | 4.188 | 81% | 61% | 1.42 |
| | Aβ ₁₋₃₈/ Aβ ₁₋₄₀ | 0.215 | 87% | 90% | 1.77 |
| FTLD versus AD | ELISA Aβ₁₋₄₂/ tau | 1.612 | 86% | 100% | 1.86 |
| | Aβ₁₋₃₈/ Aβ₁₋₄₀ | 0.215 | 87% | 87% | 1.74 |
| FTLD versus NDC and AD | ELISA Aβ₁₋₄₂/ tau | 0.978 | 100% | 45% | 1.45 |
| | Aβ₁₋₃₈/ Aβ₁₋₄₀ | 0.215 | 87% | 88% | 1.75 |

**Table 9**

| **Differential diagnosis** | **Parameter** | **Cut off** | **Sensitivity** | **Specificity** | **Youden Index** | **AUC** | **95%-CI** |
|---|---|---|---|---|---|---|---|
| AD (n=71) versus all other patients (n=232) | Aβ₁₋₄₂% | 5.57 | 87 % | 85 % | 0.72 | 0.925 | 0.897-0.954 |
| | Aβ_{1-42/ 1-38} | 0.39 | 90 % | 87% | 0.77 | 0.938 | 0.913-0.963 |
| AD (n=71) versus NDC (n=71) | Aβ₁₋₄₂% | 5.81 | 90% | 97 % | 0.87 | 0.993 | 0.984-1.00 |
| | Aβ_{1-42/ 1-38} | 0.39 | 90 % | 97 % | 0.87 | 0.989 | 0.976-1.00 |
| AD (n=71) versus all other dementias (n=161) | Aβ₁₋₄₂% | 5.55 | 85 % | 81 % | 0.66 | 0.895 | 0.856-0.935 |
| | Aβ_{1-42/ 1-38} | 0.38 | 85 % | 85 % | 0.70 | 0.915 | 0.880-0.950 |
| FTD (n=36) versus all other patients (n=267) | Aβ₁₋₃₈% | 12.7 | 86 % | 90 % | 0.76 | 0.923 | 0.868-0.978 |
| | Aβ_{1-38/1-40} | 0.21 | 86 % | 79 % | 0.65 | 0.907 | 0.855-0.960 |
| FTD (n=36) versus | Aβ₁₋₃₈% | 12.7 | 86 % | 96 % | 0.82 | 0.951 | 0.901-1.00 |
| NDC (n=71) | Aβ_{1-38/ 1-40} | 0.21 | 89 % | 87 % | 0.76 | 0.950 | 0.91-0.99 |
| FTD (n=36) versus all other dementias (n=196) | Aβ₁₋₃₈% | 12.7 | 86 % | 87 % | 0.73 | 0.913 | 0.854-0.972 |
| | Aβ_{1-38/ 1-40} | 0.21 | 86 % | 76% | 0.62 | 0.892 | 0.832-0.951 |
| PPA (n=10) versus NDC (n=71) | Aβ₁₋₃₈% | 13.54 | 80% | 75% | 0.55 | 0.866 | 0.765-0.967 |
| | Aβ_{1-38/ 1-40} | 0.22 | 70 % | 82% | 0.52 | 0.817 | 0.699-0.935 |
| PPA (n=10) versus all other dementias (n=222) | Aβ₁₋₃₈% | 13.51 | 70% | 63% | 0.33 | 0.723 | 0.625-0.821 |
| | Aβ_{1-38/ 1-40} | 0.22 | 70% | 58% | 0.28 | 0.600 | 0.459-0.740 |
| PPA (n=10) versus all other patients (n=293) | Aβ₁₋₃₈% | 13.51 | 80% | 65% | 0.45 | 0.758 | 0.663-0.852 |
| | Aβ_{1-38/ 1-40} | 0.22 | 70% | 64% | 0.34 | 0.652 | 0.521-0.783 |

| | | | | | | | |
|---|---|---|---|---|---|---|---|
| AUC: Area under curve; CI: Confidence interval | | | | | | | |

**Table 10**

| **Diagnosis** | **DC (n=7)** | **DC (n=7)** | **AD (n=7)** | **AD (n=7)** | **VAD (n=7)** | **VAD (n=7)** | **FTD (n=7)** | **FTD (n=7)** |
|---|---|---|---|---|---|---|---|---|
| | Mean | ±SD | Mean | ±SD | Mean | ±SD | Mean | ±SD |
| **age** | 64.0 | 11.11 | 67.6 | 5.71 | 71.3 | 8.06 | 63.0 | 2.94 |
| **mmse** | 27.17 | 3.6 | 19.4 | 9.61 | 18.4 | 11.01 | 24.5 | 1.91 |
| **Aβ1-37 (ng/ml)** | 0.025 | 0.003 | 0.019 | 0.006 | 0.026 | 0.006 | 0.019 | 0.006 |
| **Aβ1-38 (ng/ml)** | 0.034 | 0.008 | 0.029 | 0.011 | 0.039 | 0.007 | 0.029 | 0.007 |
| **Aβ1-39 (ng/ml)** | 0.031 | 0.005 | 0.026 | 0.007 | 0.039 | 0.005 | 0.032 | 0.010 |
| **Aβ1-40 (ng/ml)** | 0.226 | 0.051 | 0.185 | 0.087 | 0.326 | 0.091 | 0.246 | 0.078 |
| **Aβ1-42 (ng/ml)** | 0.027 | 0.004 | 0.020 | 0.007 | 0.024 | 0.006 | 0.028 | 0.011 |
| **Aβ2-40 (ng/ml)** | 0.023 | 0.002 | 0.018 | 0.005 | 0.022 | 0.004 | 0.021 | 0.006 |
| **total Aβ (ng/ml) ¹** | 0.365 | 0.065 | 0.297 | 0.122 | 0.477 | 0.100 | 0.375 | 0.113 |
| **Aβ1-37% ²** | 6.945 | 1.220 | 6.819 | 1.105 | 5.681 | 1.397 | 5.117 | 1.198 |
| **Aβ1-38% ²** | 9.269 | 0.802 | 9.896 | 0.860 | 8.239 | 0.828 | 7.877 | 0.907 |
| **Aβ1-39% ²** | 8.725 | 1.660 | 9.055 | 1.330 | 8.470 | 1.923 | 8.555 | 1.642 |
| **Aβ1-40% ²** | 61.314 | 3.353 | 60.689 | 5.492 | 67.600 | 5.329 | 65.468 | 2.124 |
| **Aβ1-42% ²** | 7.396 | 0.905 | 7.013 | 1.815 | 5.129 | 1.165 | 7.276 | 0.931 |
| **Aβ2-40% ²** | 6.350 | 1.140 | 6.528 | 2.034 | 4.880 | 1.704 | 5.707 | 0.489 |
| **Ratio Aβ1-38/1-40** | 0.151 | 0.009 | 0.164 | 0.022 | 0.123 | 0.020 | 0.121 | 0.017 |
| **Ratio Aβ1**-**38/1-42** | 1.274 | 0.218 | 1.483 | 0.360 | 1.665 | 0.354 | 1.099 | 0.194 |
| **Ratio Aβ1-40/1-42** | 8.414 | 1.218 | 9.143 | 2.239 | 13.851 | 3.689 | 9.115 | 1.120 |

| | | | | | | | | |
|---|---|---|---|---|---|---|---|---|
| ¹total Aβ peptide concentration as measured by the sum of all investigated Aβ peptides; ²percentage abundance of Aβ peptides relative to the sum of all investigated Aβ peptides. (*) and (**) indicate significant (p<0.05) and highly significant (p<0.01) alterations as compared to DC. | | | | | | | | |

## Claims

1. An *ex vivo* method of differentially diagnosing vascular dementias, comprising the step of determining in a body fluid obtained from a patient at least one relative concentration selected from the group consisting of
- the ratio of the absolute concentration of Aβ1-38 to the concentration of total amyloid β (Aβ1-38 %),
- the ratio of the absolute concentration of Aβ1-38 to the absolute concentration of amyloid β species Aβ1-40 (Aβ1-38/Aβ1-40), and
- the ratio of the absolute concentration of Aβ1-38 to the absolute concentration of amyloid β species Aβ1-42 (Aβ1-38/Aβ1-42).

2. The method of claim 1, wherein said body fluid is a blood sample.

3. The method of claim 2, wherein said blood sample is a blood plasma sample.

4. The method of any of claims 1 to 3, further comprising the step of enriching amyloid β peptides from the body fluid using an antibody to amyloid β peptide.

5. The method of any of claims 1 to 4, wherein said relative concentration of Aβ1-38 selected from the group consisting of
- Aβ1-38 %, and
- Aβ1-38/Aβ1-40,
being above a threshold value is indicative of no vascular dementia in the patient.

6. The method of any of claims 1 to 4, wherein
- Aβ1-38 % being below a threshold value or Aβ1-38/Aβ1-40 being below a threshold value, and
- the absolute concentration of Aβ1-38 being above a threshold value, or Aβ1-38/Aβ1-42 being above a threshold value, or the ratio of the absolute concentration of Aβ1-40 to the absolute concentration of amyloid β species Aβ1-42 (Aβ1-40/Aβ1-42) being above a threshold value, or the ratio of the absolute concentration of Aβ1-42 to the total concentration of amyloid β (Aβ1-42 %) being below a threshold value,
is indicative of vascular dementia in the patient.

7. The method of any of claims 1 to 4, wherein
- Aβ1-38/Aβ1-42 being above a threshold value, and
- Aβ1-40 % being above a threshold value, or Aβ1-42 % being below a threshold value, or Aβ1-40/Aβ1-42 being above a threshold value, or the absolute concentration of Aβ1-40 being above a threshold value, or the total concentration of amyloid β being above a threshold value, is indicative of vascular dementia in the patient.

8. Use of components for determining the concentration of Aβ1-38 in a body fluid for the *ex vivo* differential diagnosis of vascular dementias.

9. The use according to claim 8 wherein said components for determining the concentration of Aβ1-38 are antibodies.

10. The use according to claim 8 or 9, further comprising the use of an antibody to amyloid β peptides for enriching amyloid β peptides from the body fluid.

11. The use according to any one of claims 8 to 10, wherein said components for determining the concentration of Aβ1-38 allow to determine the concentration of Aβ1-38 as a relative concentration.

12. The use according to any one of claims 8 to 11, wherein said components for determining the concentration of Aβ1-38 allow to determine at least one relative concentration of Aβ1-38 selected from the group consisting of
- the ratio of the absolute concentration of Aβ1-38 to the concentration of total amyloid β peptide in the body fluid (Aβ1-38 %),
- the ratio of the absolute concentration of Aβ1-38 to the absolute concentration of the amyloid β peptide species Aβ1-40 in the body fluid (Aβ1-38/Aβ1-40), and
- the ratio of the absolute concentration of Aβ1-38 to the absolute concentration of the amyloid β peptide species Aβ1-42 in the body fluid (Aβ1-38/Aβ1-42).

13. The use according to any of claims 8 to 12, comprising the use of components for determining at least one further concentration selected from the group consisting of:
- the absolute concentration of Aβ1-38,
- the absolute concentration of Aβ1-40,
- the ratio of the absolute concentration of Aβ1-40 to the total concentration of amyloid β (Aβ1-40 %),
- the ratio of the absolute concentration of Aβ1-40 to the absolute concentration of amyloid β species Aβ1-42 (Aβ1-40/Aβ1-42),
- the absolute concentration of Aβ1-42,
- the ratio of the absolute concentration of Aβ1-42 to the total concentration of amyloid β (Aβ1-42 %), and
- the total concentration of amyloid β.

14. The use according to any of claims 8 to 13, comprising the use of components for indicating that the concentration selected from the group consisting of:
- the absolute concentration of Aβ1-38,
- the ratio of the absolute concentration of Aβ1-38 to the total concentration of amyloid β (Aβ1-38 %),
- the ratio of the absolute concentration of Aβ1-38 to the absolute concentration of amyloid β species Aβ1-40 (Aβ1-38/Aβ1-40),
- the ratio of the absolute concentration of Aβ1-38 to the absolute concentration of amyloid β species Aβ1-42 (Aβ1-38/Aβ1-42),
- the absolute concentration of Aβ1-40,
- the ratio of the absolute concentration of Aβ1-40 to the total concentration of amyloid β (Aβ1-40 %),
- the ratio of the absolute concentration of Aβ1-40 to the absolute concentration of amyloid β species Aβ1-42 (Aβ1-40/Aβ1-42),
- the absolute concentration of Aβ1-42,
- the ratio of the absolute concentration of Aβ1-42 to the total concentration of amyloid β (Aβ1-42 %), and
- the total concentration of amyloid β,
is above or below a threshold value.

15. The use according to claim 13 or 14, wherein said components for determining at least one further concentration are antibodies.

## Patentansprüche

1. Ein ex vivo-Verfahren zum differentiellen Diagnostizieren vaskulärer Demenzen, wobei das Verfahren den Schritt des Bestimmens mindestens einer relativen Konzentration in einer Körperflüssigkeit, welche von einem Patienten erhalten wurde, aufweist, wobei die relative Konzentration aus der Gruppe ausgewählt ist, welche besteht aus
- dem Verhältnis der absoluten Konzentration von Aβ1-38 zu der Gesamtkonzentration von Amyloid β (Aβ1-38 %),
- dem Verhältnis der absoluten Konzentration von Aβ1-38 zur absoluten Konzentration von Amyloid β-Spezies Aβ1-40 (Aβ1-38/Aβ1-40) und
- dem Verhältnis der absoluten Konzentration von Aβ1-38 zur absoluten Konzentration von Amyloid β-Spezies Aβ1-42 (Aβ1-38/Aβ1-42).

2. Das Verfahren nach Anspruch 1, wobei die Körperflüssigkeit eine Blutprobe ist

3. Das Verfahren nach Anspruch 2, wobei die Blutprobe eine Blutplasmaprobe ist.

4. Das Verfahren nach einem der Ansprüche 1 bis 3, das weiterhin den Schritt des Anreicherns von Amyloid β-Peptiden von der Körperflüssigkeit unter Verwendung eines Antikörpers gegen Amyloid β-Peptid aufweist.

5. Das Verfahren nach einem der Ansprüche 1 bis 4, wobei die aus der aus
- Aβ1-38 % und
- Aβ1-38/Aβ1-40
bestehenden Gruppe ausgewählte Relativkonzentration von Aβ1-38 oberhalb eines Grenzwerts liegend ein Hinweis auf keine vaskuläre Demenz bei dem Patienten ist.

6. Das Verfahren nach einem der Ansprüche 1 bis 4, wobei
- Aβ1-38 % oberhalb eines Grenzwerts liegend oder Aβ1-38/Aβ1-40 unterhalb eines Grenzwerts liegend und
- die absolute Konzentration von Aβ1-38 oberhalb eines Grenzwerts liegend oder Aβ1-38/Aβ1-42 oberhalb eines Grenzwerts liegend oder das Verhältnis der absoluten Konzentration von Aβ1-40 zu der absoluten Konzentration der Amyloid β-Spezies Aβ1-42 (Aβ1-40/Aβ1-42) oberhalb eines Grenzwerts liegend oder das Verhältnis der absoluten Konzentration von Aβ1-42 zu der Gesamtkonzentration von Amyloid β (Aβ1-42 %) unterhalb eines Grenzwerts liegend
ein Hinweis auf vaskuläre Demenz bei dem Patienten ist.

7. Das Verfahren nach einem der Ansprüche 1 bis 4, wobei
- Aβ1-38/Aβ1-42 oberhalb eines Grenzwerts liegend und
- Aβ1-40 % oberhalb eines Grenzwerts liegend oder Aβ1-42 % unterhalb eines Grenzwerts liegend oder Aβ1-40/A-42 oberhalb eines Grenzwerts liegend oder die absolute Konzentration von Aβ1-40 oberhalb eines Grenzwerts liegend oder die absolute Konzentration von Amyloid β oberhalb eines Grenzwerts liegend
ein Hinweis auf vaskuläre Demenz bei dem Patienten ist.

8. Verwendung von Komponenten zum Bestimmen der Konzentration von Aβ1-38 in einer Körperflüssigkeit für die differentielle ex vivo-Diagnostik von vaskulären Demenzen.

9. Die Verwendung nach Anspruch 8, wobei die Komponenten zum Bestimmen der Konzentration von Aβ1-38 Antikörper sind.

10. Die Verwendung nach Anspruch 8 oder 9, die weiterhin die Verwendung eines Antikörpers gegen Amyloid β-Peptide zum Anreichern von Amyloid β-Peptiden von der Körperflüssigkeit aufweist.

11. Die Verwendung nach einem der Ansprüche 8 bis 10, wobei es die Komponenten zum Bestimmen der Konzentration von Aβ1-38 ermöglichen, die Konzentration von Aβ1-38 als relative Konzentration zu bestimmen.

12. Die Verwendung nach einem der Ansprüche 8 bis 11, wobei es die Komponenten zum Bestimmen der Konzentration von Aβ1-38 ermöglichen, mindestens eine relative Konzentration von Aβ1-38 zu bestimmen, die aus der Gruppe ausgewählt ist, welche besteht aus
- dem Verhältnis der absoluten Konzentration von Aβ1-38 zu der Konzentration des gesamten Amyloid β-Peptids in der Körperflüssigkeit (Aβ1-38 %),
- dem Verhältnis der absoluten Konzentration von Aβ1-38 zu der absoluten Konzentration der Amyliod β-Peptidspezies Aβ1-40 in der Körperflüssigkeit (Aβ1-38/Aβ1-40) und
- dem Verhältnis der absoluten Konzentration von Aβ1-38 zu der absoluten Konzentration der Amyloid β-Peptidspezies Aβ1-42 in der Körperflüssigkeit (Aβ1-38/Aβ1-42).

13. Die Verwendung nach einem der Ansprüche 8 bis 12, die die Verwendung von Komponenten zum Bestimmen mindestens einer weiteren Konzentration aufweist, welche aus der Gruppe ausgewählt ist, die besteht aus
- der absoluten Konzentration von Aβ1-38,
- der absoluten Konzentration von Aβ1-40,
- dem Verhältnis der absoluten Konzentration von Aβ1-40 zu der Gesamtkonzentration von Amyloid β (Aβ1-40 %),
- dem Verhältnis der absoluten Konzentration von Aβ1-40 zu der absoluten Konzentration von Amyloid β-Spezies Aβ1-42 (Aβ1-40/ Aβ1-42),
- der absoluten Konzentration von Aβ1-42,
- dem Verhältnis der absoluten Konzentration von Aβ1-42 zu der Gesamtkonzentration von Amyloid β (Aβ1-42 %) und
- der Gesamtkonzentration von Amyloid β.

14. Die Verwendung nach einem der Ansprüche 8 bis 13, die die Verwendung von Komponenten zum Anzeigen aufweist, dass die Konzentration, welche aus der Gruppe ausgewählt ist, die besteht aus:
- der absoluten Konzentration von Aβ1-38,
- dem Verhältnis der absoluten Konzentration von Aβ1-38 zu der Gesamtkonzentration von Amyloid β (Aβ1-38 %),
- dem Verhältnis der absoluten Konzentration von Aβ1-38 zu der absoluten Konzentration von Amyloid β-Spezies Aβ1-40 (Aβ1-38/Aβ1-40),
- dem Verhältnis der absoluten Konzentration von Aβ1-38 zu der absoluten Konzentration von Amyloid β Spezies Aβ1-42 (Aβ1-38/ Aβ1-42),
- der absoluten Konzentration von Aβ1-40,
- dem Verhältnis der absoluten Konzentration von Aβ1-40 zu der Gesamtkonzentration von Amyloid β (Aβ1-40 %),
- dem Verhältnis der absoluten Konzentration von Aβ1-40 zu der absoluten Konzentration von Amyloid β Spezies Aβ1-42 (Aβ1-40/ Aβ1-42),
- der absoluten Konzentration von Aβ1-42,
- dem Verhältnis der absoluten Konzentration von Aβ1-42 zu der Gesamtkonzentration von Amyloid β (Aβ1-42 %) und
- der Gesamtkonzentration von Amyloid β,
oberhalb oder unterhalb eines Grenzwerts liegt.

15. Die Verwendung nach Anspruch 13 oder 14, wobei die Komponenten zum Bestimmen der mindestens einen weiteren Konzentration Antikörper sind.

## Revendications

1. Procédé *ex vivo* pour établir un diagnostic différentiel de démences vasculaires, comprenant l'étape qui consiste à déterminer dans un fluide corporel obtenu chez un patient au moins une concentration relative sélectionnée dans le groupe consistant en
- le rapport de la concentration absolue d'Aβ1-38 sur la concentration de β-amyloïde totale (% d'Aβ1-38),
- le rapport de la concentration absolue d'Aβ1-38 sur la concentration absolue de l'espèce de β-amyloïde Aβ1-40 (Aβ1-38/Aβ1-40), et
- le rapport de la concentration absolue d'Aβ1-38 sur la concentration absolue de l'espèce de β-amyloïde Aβ1-42 (Aβ1-38/Aβ1-42).

2. Procédé selon la revendication 1, dans lequel ledit fluide corporel est un échantillon de sang.

3. Procédé selon la revendication 2, dans lequel ledit échantillon de sang est un échantillon de plasma sanguin.

4. Procédé selon l'une quelconque des revendications 1 à 3, comprenant en outre l'étape qui consiste à réaliser un enrichissement en peptides β-amyloïde à partir du fluide corporel en utilisant un anticorps dirigé contre un peptide β-amyloïde.

5. Procédé selon l'une quelconque des revendications 1 à 4, dans lequel ladite concentration relative d'Aβ1-38 sélectionnée dans le groupe consistant en
- le % d'Aβ1-38, et
- Aβ1-38/Aβ1-40,
qui est au-dessus d'une valeur seuil indique l'absence de démence vasculaire chez le patient.

6. Procédé selon l'une quelconque des revendications 1 à 4, dans lequel
- le % d'Aβ1-38 qui est en dessous d'une valeur seuil ou Aβ1-38/Aβ1-40 qui est en dessous d'une valeur seuil, et
- la concentration absolue d'Aβ1-38 qui est au-dessus d'une valeur seuil, ou Aβ1-38/Aβ1-42 qui est au-dessus d'une valeur seuil, ou le rapport de la concentration absolue d'Aβ1-40 sur la concentration absolue de l'espèce de β-amyloïde Aβ1-42 (Aβ1-40/Aβ1-42) qui est au-dessus d'une valeur seuil, ou le rapport de la concentration absolue d'Aβ1-42 sur la concentration totale de β-amyloïde (% d'Aβ1-42) qui est en dessous d'une valeur seuil,
indique une démence vasculaire chez le patient.

7. Procédé selon l'une quelconque des revendications 1 à 4, dans lequel
- Aβ1-38/Aβ1-42 qui est au-dessus d'une valeur seuil, et
- le % d'Aβ1-40 qui est au-dessus d'une valeur seuil, ou le % d'Aβ1-42 qui est en dessous d'une valeur seuil, ou Aβ1-40/Aβ1-42 qui est au-dessus d'une valeur seuil, ou la concentration absolue d'Aβ1-40 qui est au-dessus d'une valeur seuil, ou la concentration totale de β-amyloïde qui est au-dessus d'une valeur seuil, indique une démence vasculaire chez le patient.

8. Utilisation de composants pour déterminer la concentration d'Aβ1-38 dans un fluide corporel pour établir le diagnostic différentiel ex *vivo* de démences vasculaires.

9. Utilisation selon la revendication 8, dans laquelle lesdits composants pour déterminer la concentration d'Aβ1-38 sont des anticorps.

10. Utilisation selon la revendication 8 ou 9, comprenant en outre l'utilisation d'un anticorps dirigé contre des peptides β-amyloïdes afin d'obtenir un enrichissement en peptides β-amyloïdes à partir du fluide corporel.

11. Utilisation selon l'une quelconque des revendications 8 à 10, dans laquelle lesdits composants pour déterminer la concentration d'Aβ1-38 permettent de déterminer la concentration d'Aβ1-38 en tant que concentration relative.

12. Utilisation selon l'une quelconque des revendications 8 à 11, dans laquelle lesdits composants pour déterminer la concentration d'Aβ1-38 permettent de déterminer au moins une concentration relative d'Aβ1-38 sélectionnée dans le groupe consistant en
- le rapport de la concentration absolue d'Aβ1-38 sur la concentration de peptide β-amyloïde total dans le fluide corporel (% d'Aβ1-38),
- le rapport de la concentration absolue d'Aβ1-38 sur la concentration absolue de l'espèce de peptide β-amyloïde Aβ1-40 dans le fluide corporel (Aβ1-38/Aβ1-40), et
- le rapport de la concentration absolue d'Aβ1-38 sur la concentration absolue de l'espèce de peptide β-amyloïde Aβ1-42 dans le fluide corporel (Aβ1-38/Aβ1-42).

13. Utilisation selon l'une quelconque des revendications 8 à 12, comprenant l'utilisation de composants pour déterminer au moins une concentration supplémentaire sélectionnée dans le groupe consistant en :
- la concentration absolue d'Aβ1-38,
- la concentration absolue d'Aβ1-40,
- le rapport de la concentration absolue d'Aβ1-40 sur la concentration totale de β-amyloïde (% d'Aβ1-40),
- le rapport de la concentration absolue d'Aβ1-40 sur la concentration absolue de l'espèce de β-amyloïde Aβ1-42 (Aβ1-40/Aβ1-42),
- la concentration absolue d'Aβ1-42,
- le rapport de la concentration absolue d'Aβ1-42 sur la concentration totale de β-amyloïde (% d'Aβ1-42), et
- la concentration totale de β-amyloïde.

14. Utilisation selon l'une quelconque des revendications 8 à 13, comprenant l'utilisation de composants pour indiquer que la concentration sélectionnée dans le groupe consistant en :
- la concentration absolue d'Aβ1-38,
- le rapport de la concentration absolue d'Aβ1-38 sur la concentration totale de β-amyloïde (% d'Aβ1-38),
- le rapport de la concentration absolue d'Aβ1-38 sur la concentration absolue de l'espèce de β-amyloïde Aβ1-40 (Aβ1-38/Aβ1-40),
- le rapport de la concentration absolue d'Aβ1-38 sur la concentration absolue de l'espèce de β-amyloïde Aβ1-42 (Aβ1-38/Aβ1-42),
- la concentration absolue d'Aβ1-40,
- le rapport de la concentration absolue d'Aβ1-40 sur la concentration totale de β-amyloïde (% d'Aβ1-40),
- le rapport de la concentration absolue d'Aβ1-40 sur la concentration absolue de l'espèce de β-amyloïde Aβ1-42 (Aβ1-40/Aβ1-42),
- la concentration absolue d'Aβ1-42,
- le rapport de la concentration absolue d'Aβ1-42 sur la concentration totale de β-amyloïde (% d'Aβ1-42), et
- la concentration totale de β-amyloïde,
est au-dessus ou en dessous d'une valeur seuil.

15. Utilisation selon la revendication 13 ou 14, dans laquelle lesdits composants pour déterminer au moins une concentration supplémentaire sont des anticorps.
